(19) Europäisches Patentamt
European Patent Office
Office européen des brevets

(11) Veröffentlichungsnummer: **0 380 000 A2**

# (12) EUROPÄISCHE PATENTANMELDUNG

(21) Anmeldenummer: **90101129.6**

(51) Int. Cl.5: **C07D 207/34, C07D 405/06**

(22) Anmeldetag: **20.01.90**

(30) Priorität: **26.01.89 DE 3902216**

(43) Veröffentlichungstag der Anmeldung:
**01.08.90 Patentblatt 90/31**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Pfister, Juergen, Dr.**
**Ziegelofenweg 1**
**D-6720 Speyer(DE)**
Erfinder: **Wiesenfeldt, Matthias, Dr.**
**Rosenstrassse 10**
**D-6704 Mutterstadt(DE)**
Erfinder: **Etzbach, Karl-Heinz, Dr.**
**Carl-Bosch-Ring 55**
**D-6710 Frankenthal(DE)**

## (54) Pyrrolderivate.

(57) Pyrrolderivate der Formel

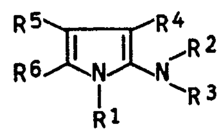

oder deren Tautomere, in der
$R^1$ Wasserstoff, Alkyl, Alkenyl, Alkinyl, Cycloalkyl, Phenyl, Hetaryl oder Amino, wobei diese Reste substituiert sein können,
$R^2$ Wasserstoff, Alkyl, Benzyl, Alkenyl, Cycloalkyl oder Phenyl,
$R^3$ Wasserstoff, Alkyl, Benzyl oder Alkenyl oder $R^2$ und $R^3$ zusammen einen Rest der Formel

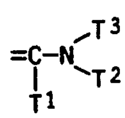 ,

wobei $T^1$, $T^2$ und $T^3$ die in der Beschreibung genannte Bedeutung besitzen, oder $R^2$ und $R^3$ zusammen mit dem sie verbindenden Stickstoffatom einen heterocyclischen Rest,
$R^4$ Cyano, gegebenenfalls substituiertes Carbamoyl oder Thiocarbamoyl oder den Rest $-C(NH_2)=N-OH$,
$R^5$ Halogen, Hydroxy, Alkanoyloxy oder Benzoyloxy und
$R^6$ Wasserstoff, Alkyl, Phenyl, Cyano, Halogen, Nitro, Hydroxysulfonyl, Alkanoyl, Benzoyl oder einen Rest der Formel

$-CH=NOH$ oder 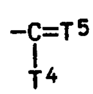 ,

EP 0 380 000 A2

wobei $T^4$ für Alkyl oder Phenyl und $T^5$ für den Rest einer methylenaktiven Verbindung, Hydroxyimino oder den Rest eines primären Amins stehen,
bedeuten, mit der Maßgabe, daß $R^1$, $R^2$ und $R^3$ nicht gleichzeitig Wasserstoff bedeuten, ein Verfahren zur Herstellung von Pyrrolderivaten (mit $R^5$ = Hydroxy) sowie die Verwendung der neuen Produkte als Diazo- oder Kupplungskomponenten.

## Pyrrolderivate

Die Erfindung betrifft Verbindungen der allgemeinen Formel I

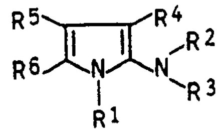

(I)

oder deren Tautomere, in der

$R^1$ Wasserstoff, $C_1$-$C_{20}$-Alkyl, das gegebenenfalls substituiert und/oder durch ein oder mehrere Sauerstoffatome unterbrochen ist, gegebenenfalls substituiertes $C_3$-$C_6$-Alkenyl, gegebenenfalls substituiertes $C_3$-$C_6$-Alkinyl, gegebenenfalls substituiertes $C_3$-$C_{10}$-Cycloalkyl, gegebenenfalls substituiertes Phenyl, Pyridyl, Thienyl, Amino, $C_1$-$C_4$-Alkanoylamino, Benzoylamino, $C_1$-$C_4$-Dialkylamino oder den Rest

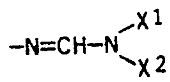

,

wobei $X^1$ für $C_1$-$C_4$-Alkyl oder Phenyl und $X^2$ für $C_1$-$C_4$-Alkyl stehen,

$R^2$ Wasserstoff, $C_1$-$C_4$-Alkyl, Benzyl, $C_3$-$C_6$-Alkenyl, $C_5$-$C_7$-Cycloalkyl oder gegebenenfalls substituiertes Phenyl,

$R^3$ Wasserstoff, $C_1$-$C_4$-Alkyl, Benzyl oder $C_3$-$C_6$-Alkenyl oder $R^2$ und $R^3$ zusammen einen Rest der Formel

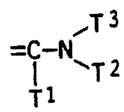

,

in dem $T^1$ für Wasserstoff, $C_1$-$C_4$-Alkyl oder Phenyl, $T^2$ für $C_1$-$C_4$-Alkyl oder gegebenenfalls substituiertes Phenyl und $T^3$ für $C_1$-$C_4$-Alkyl stehen, oder $R^2$ und $R^3$ zusammen mit dem sie verbindenden Stickstoffatom einen 5- bis 7-gliedrigen gesättigten heterocyclischen Rest, der gegebenenfalls weitere Heteroatome enthält,

$R^4$ Cyano, Carbamoyl, $C_1$-$C_4$-Mono- oder Dialkylcarbamoyl, Thiocarbamoyl, $C_1$-$C_4$-Mono- oder Dialkylthiocarbamoyl oder den Rest

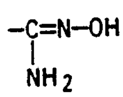

,

$R^5$ Halogen, Hydroxy, $C_1$-$C_{20}$-Alkanoyloxy oder gegebenenfalls substituiertes Benzoyloxy und

$R^6$ Wasserstoff, $C_1$-$C_4$-Alkyl, gegebenenfalls substituiertes Phenyl, Cyano, Halogen, Nitro, Hydroxysulfonyl, $C_1$-$C_{10}$-Alkanoyl, gegebenenfalls substituiertes Benzoyl oder einen Rest der Formel

$-CH{=}N{-}OH$ oder 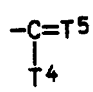 ,

bedeuten, wobei $T^4$ für $C_1$-$C_4$-Alkyl oder gegebenenfalls substituiertes Phenyl und $T^5$ für den Rest einer methylenaktiven Verbindung, Hydroxyimino oder den Rest N-$X^3$ stehen, in dem $X^3$ die Bedeutung von $C_1$-$C_{20}$-Alkyl, das gegebenenfalls substituiert und/oder durch ein oder mehrere Sauerstoffatome unterbrochen ist, gegebenenfalls substituiertem $C_3$-$C_6$-Alkenyl, gegebenenfalls substituiertem $C_3$-$C_6$-Alkinyl, gegebenenfalls substituiertem $C_3$-$C_{10}$-Cycloalkyl, gegebenenfalls substituiertem Phenyl, Pyridyl, $C_1$-$C_4$-Alkoxycarbonylmethyl, Amino, $C_1$-$C_4$-Dialkylamino oder Phenylamino besitzt, mit der Maßgabe, daß $R^1$, $R^2$ und $R^3$ nicht

gleichzeitig Wasserstoff bedeuten.

Aus der DD-A-126 389 ist 5-Methyl- und 5-Ethyl-2-amino-3-cyanopyrrolin-4-on bekannt. Diese Verbindungen sind als Diazokomponenten für die Herstellung von Azofarbstoffen jedoch nicht besonders geeignet.

Aufgabe der vorliegenden Erfindung war es nun, neue Pyrrolderivate bereitzustellen, die sich je nach Konstitution sowohl als Diazokomponenten als auch als Kupplungskomponenten eignen.

Demgemäß wurden die eingangs näher bezeichneten Pyrrolderivate der Formel I gefunden.

Alle in der obengenannten Formel I auftretenden Alkyl- und Alkenylgruppen können sowohl geradkettig als auch verzweigt sein.

Wenn in der Formel I Alkylgruppen auftreten, die durch ein oder mehrere Sauerstoffatome unterbrochen sind, sind solche Alkylgruppen bevorzugt, die durch ein bis drei, insbesondere ein bis zwei Sauerstoffatome unterbrochen sind.

Wenn in der Formel I substituierte Phenylgruppen auftreten, können als Substituenten z.B. $C_1$-$C_4$-Alkyl, $C_1$-$C_4$-Alkoxy, Halogen, dabei insbesondere Chlor oder Brom, Nitro, Hydroxy oder Amino in Betracht kommen.

Wenn in der Formel I substituierte Alkylgruppen auftreten, können als Substituenten z.B. $C_1$-$C_5$-Alkylthio, gegebenenfalls substituiertes Phenoxy, Halogen, dabei insbesondere Chlor oder Brom, Hydroxy, Amino, $C_1$-$C_4$-Mono-oder Dialkylamino, Pyrrolidino, Piperidino, Morpholino, Thiomorpholino, Piperazino, N-($C_1$-$C_4$-Alkyl)piperazino, Hexamethylenimino, $C_1$-$C_5$-Alkoxycarbonyl oder gegebenenfalls substituiertes Phenyl in Betracht kommen.

Wenn in der Formel I substituiertes Alkenyl-, Alkinyl- oder Cycloalkylgruppen auftreten, können als Substituenten z.B. Fluor, Chlor oder Brom in Betracht kommen.

Wenn die Reste $R^2$ und $R^3$ zusammen mit dem sie verbindenden Stickstoffatom einen 5- bis 7-gliedrigen gesättigten heterocyclischen Rest, der gegebenenfalls weitere Heteroatome enthält, bedeuten, so können dafür z.B. Pyrrolidino, Piperidino, Morpholino, Thiomorpholino, Thiomorpholino-S,S-dioxid, Piperazino, N-($C_1$-$C_4$-Alkyl)piperazino oder Hexamethylenimino in Betracht kommen.

Reste $R^1$ sind zum Beispiel Wasserstoff, Methyl, Ethyl, Propyl, Isopropyl, Butyl, Isobutyl, sec-Butyl, Pentyl, Isopentyl, Neopentyl, tert-Pentyl, Hexyl, Heptyl, Octyl, Isooctyl, 2-Ethylhexyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl, Eicosyl, Allyl, Propinyl, Methallyl, Cyclopropyl, Cyclobutyl, Cyclopentyl, Cyclohexyl, Cycloheptyl, Cyclooctyl, Cyclononyl, Cyclodecyl, 2-Hydroxyethyl, 2-Methoxyethyl, 2-Ethoxyethyl, 2- oder 3-Hydroxypropyl, 2- oder 3-Methoxypropyl, 2- oder 3-Ethoxypropyl, 2- oder 3-(2-Phenoxyethoxy)propyl, 2- oder 3-Benzyloxypropyl, 2-Methylthioethyl, 2-Ethylthioethyl, 2- oder 3-Methylthiopropyl, 2- oder 3-Ethylthiopropyl, 2-Aminoethyl, 2-(N,N-Dimethylamino)ethyl, 2-(N,N-Diethylamino)ethyl, 2- oder 3-(N,N-Dimethylamino)propyl, 2- oder 3-(N,N-Diethylamino)propyl, 2- oder 3-Aminopropyl, Benzyl, 1-Phenylethyl, 2-Phenylethyl, Phenyl, 2-Hydroxyphenyl, 3-Hydroxyphenyl, 4-Hydroxyphenyl, 2-Methoxyphenyl, 3-Methoxyphenyl, 4-Methoxyphenyl, 2-Ethoxyphenyl, 3-Ethoxyphenyl, 4-Ethoxyphenyl, 2-Chlorphenyl, 3-Chlorphenyl, 4-Chlorphenyl, 2-Nitrophenyl, 3-Nitrophenyl, 4-Nitrophenyl, 2-Aminophenyl, 3-Aminophenyl, 4-Aminophenyl, 2-Methylphenyl, 3-Methylphenyl, 4-Methylphenyl, 2-Ethylphenyl, 3-Ethylphenyl, 4-Ethylphenyl, Pyrid-2-yl, Pyrid-3-yl, Pyrid-4-yl, Methoxycarbonylmethyl, Ethoxycarbonylmethyl, Propoxycarbonylmethyl, Butoxycarbonylmethyl, 2-Methoxycarbonylethyl, 2-Ethoxycarbonylethyl, 2-Propoxycarbonylethyl, 2-Butoxycarbonylethyl, 2-(3,4-Dichlorphenyl)ethyl, Diphenylmethyl, 2-(3-Chlor-2-methylphenyl)ethyl, 2-(3,4-Dimethoxyphenyl)ethyl, 2-(2-Chlorphenyl)ethyl, Amino, Dimethylamino, Diethylamino, Thien-2-yl, 2-Morpholinoethyl, 2- oder 3-Morpholinopropyl oder 2-Piperazinoylethyl. (Die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Enzyklopädie der technischen Chemie, 4. Auflage, Band 7, Seiten 215 bis 217, sowie Band 11, Seiten 435 und 436.)

Reste $R^2$ und $R^3$ sind beispielsweise Methyl, Ethyl, Propyl, Isopropyl, Butyl, Isobutyl, sec-Butyl oder Allyl.

Reste $R^5$ sind beispielsweise Chlor, Brom, Acetyloxy, Propionyloxy, Butyryloxy, Isobutyryloxy, Hexanoyloxy oder Benzoyloxy.

Reste $R^6$ sind beispielsweise Methyl, Ethyl, Formyl, Acetyl, Propionyl, Butyryl, Isobutyryl, Pentanoyl, Hexanoyl, Benzoyl, Chlor oder Brom.

Der Rest $-NX^3$ leitet sich von primären Aminen der Formel $H_2NX^3$ ab. Als solche sind beispielsweise Methylamin, Ethylamin, Propylamin, Isopropylamin, Butylamin, Isobutylamin, sec-Butylamin, tert-Butylamin, Pentylamin, Isopentylamin, Neopentylamin, Hexylamin, Heptylamin, n-Octylamin, Isooctylamin, 2-Ethylhexylamin, Nonylamin, Isononylamin, Decylamin, Isodecylamin, Undecylamin, Dodecylamin, Tridecylamin, Isotridecylamin, Tetradecylamin, Pentadecylamin, Hexadecylamin, Heptadecylamin, Octadecylamin, Nonadecylamin, Eicosylamin, Allylamin, Methallylamin, Propargylamin, Cyclopropylamin, Cyclobutylamin, Cyclopentylamin, Cyclohexylamin, Cycloheptylamin, Cyclooctylamin, Cyclononylamin, Cyclodecylamin, 2-Hydroxyethylamin, 2-Methoxyethylamin, 2-Ethoxyethylamin, 3-Hydroxypropylamin, 3-Methoxypropylamin, 3-Ethoxypropylamin, 3-(2-Phenoxyethoxy)propylamin, 3-Benzyloxypropylamin, 2-(N,N-Dimethylamino)-ethylamin, 2-(N,N-Diethylamino)ethylamin, 3-(N,N-Dimethylamino)propylamin, 3-(N,N-Diethylamino)-propylamin, Benzylamin, 2-Phenylethylamin, 3-Phenylpropylamin, Anilin, 2-Hydroxyanilin, 3-Hydroxyanilin, 4-Hydroxyanilin, o-Anisidin, m-Anisidin, p-Anisidin, o-Phenetidin, m-Phenetidin, p-Phenetidin, 2-Chloranilin, 3-Chloranilin, 3-Nitroanilin, 4-Nitroanilin, o-Toluidin, m-Toluidin, p-Toluidin, 1,2-Phenylendiamin, 1,3-Phenylendiamin, 1,4-Phenylendiamin, 2-Ethylanilin, 3-Ethylanilin, 4-Ethylanilin, 2-Aminopyridin, 3-Aminopyridin, 4-Aminopyridin, Glycinmethylester, Glycinethylester, Glycinpropylester, Glycinbutylester, Hydrazin, N,N-Dimethylhydrazin oder Phenylhydrazin zu nennen.

Der Rest $T^5$ leitet sich von methylenaktiven Verbindungen der Formel $H_2T^5$ ab. Solche Verbindungen gehorchen beispielsweise der Formel

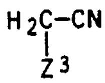
,

in der $Z^3$ Wasserstoff, Cyano, Nitro, $C_1$-$C_6$-Alkanoyl, Benzoyl, $C_1$-$C_4$-Alkylsulfonyl, Phenylsulfonyl, Carboxyl, $C_1$-$C_4$-Alkoxycarbonyl, Carbamoyl oder $C_1$-$C_4$-Mono- oder Dialkylcarbamoyl bedeutet, oder der Formel

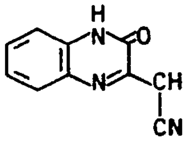, 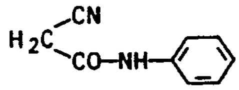, 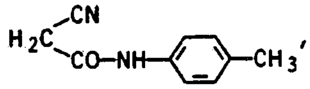, 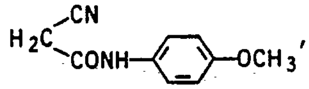,

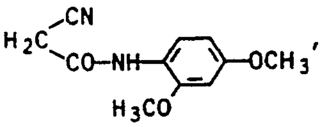, 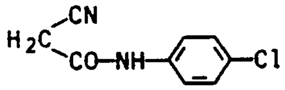, 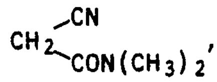, 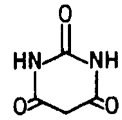,

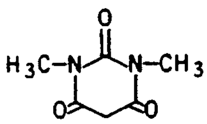, 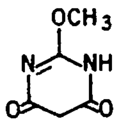, 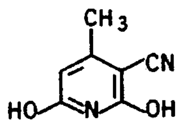, 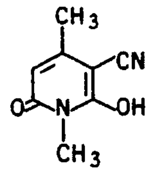, 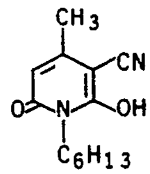,

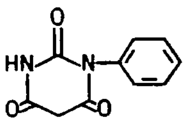, 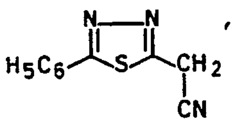, 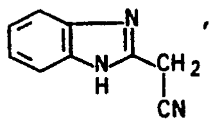, 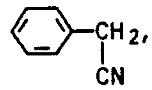, 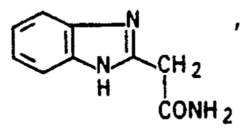,

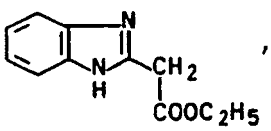, 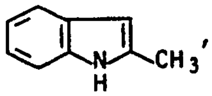, 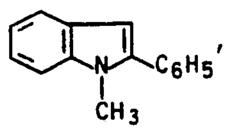, 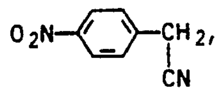,

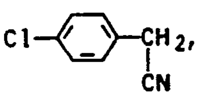, 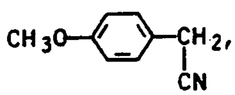, 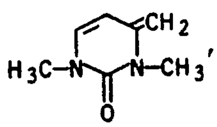, $CH_3-NO_2$, $C_2H_5-NO_2$,

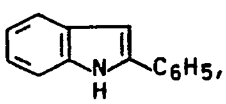, 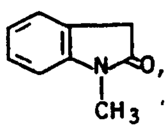, 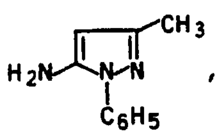, 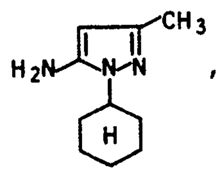, 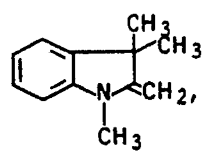,

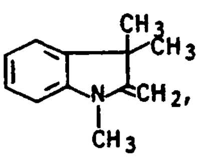, 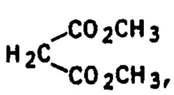, 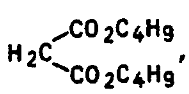, 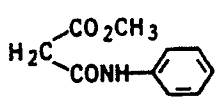 oder 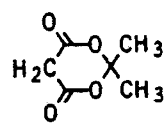

Einzelne besonders wichtige Verbindungen sind z.B.:

$H_2C(CN)_2$, 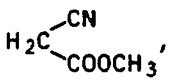, 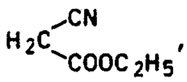, 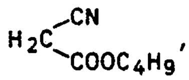, 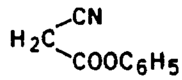, 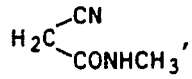,

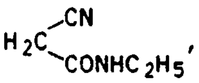, 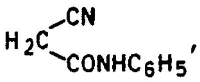, 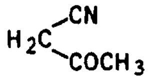 oder 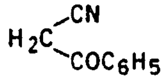 sowie 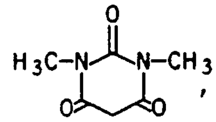,

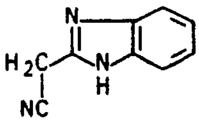, 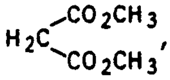, 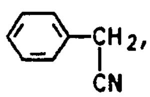, 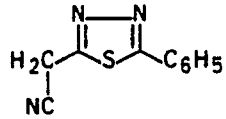 oder 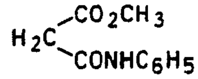.

Die Herstellung von Pyrrolderivaten der Formel Ia oder Ib

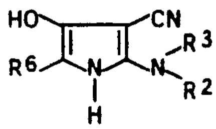 (Ia) 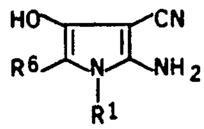 (Ib) ,

wobei $R^1$, $R^2$, $R^3$ und $R^6$ jeweils die obengenannte Bedeutung besitzen, erfolgt vorteilhaft durch Umsetzung der Verbindung der Formel II

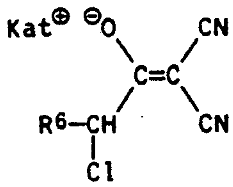 (II),

in der $R^6$ die obengenannte Bedeutung besitzt und $Kat^{\oplus}$ ein Kation (z.B. ein Alkaliion oder vorzugsweise ein Trialkylammoniumion) bedeutet, mit einem Amin der Formel IIIa oder IIIb

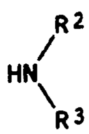 (IIIa) $R^1{-}NH_2$ (IIIb),

wobei $R^1$, $R^2$ und $R^3$ jeweils die obengenannte Bedeutung besitzen.

Die Umsetzung wird zweckmäßig in einem inerten Lösungsmittel (z.B. N,N-Dimethylformamid, N,N-Dimethylacetamid, N-Methylpyrrolidinon, Hexamethylphosphorsäuretrisamid, 1,3-Dimethylimidazolidin-2-on, 1,3-Dimethylhexahydropyrimidin-2-on oder 1,2-Diethoxyethan) in Gegenwart einer Base (z.B. Triethylamin, Tripropylamin, Tributylamin, N-Methylpiperidin oder N,N-Dimethylanilin) bei einer Temperatur von 0 bis 150 °C durchgeführt. Das Molverhältnis Verbindung III : Amin beträgt in der Regel 1:1 bis 1:5.

Die Verbindung der Formel II kann auf an sich bekanntem Wege aus Malodinitril und dem Carbonsäurechlorid der Formel IV

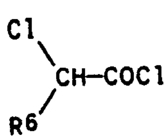 (IV),

in der $R^6$ die obengenannte Bedeutung besitzt, hergestellt werden.

Die Pyrrolderivate der Formel Ia und Ib können dann nach literaturbekannten Methoden (z.B. mittels Vilsmeier-Reaktion) zu weiteren Verbindungen der Formel I umgesetzt werden.

Weitere Einzelheiten der Herstellung können den Beispielen entnommen werden.

Die neuen Pyrrolderivate der Formel I eignen sich je nach Konstitution als Diazo- oder Kupplungskomponenten für Azofarbstoffe.

Bevorzugt sind Pyrrolderivate der Formel I in der

$R^1$ Wasserstoff, $C_1$-$C_{15}$-Alkyl, $C_5$-$C_7$-Cycloalkyl, $C_3$-$C_5$-Alkenyl, $C_2$-$C_5$-Hydroxyalkyl, $C_2$-$C_5$-Halogenalkyl, $C_1$-$C_5$-Alkoxy-$C_2$-$C_5$-alkyl, Phenoxy-$C_2$-$C_5$-alkoxy-$C_2$-$C_5$-alkyl, Phenyl-$C_1$-$C_5$-alkoxy-$C_2$-$C_5$-alkoxy-$C_2$-$C_5$-alkyl, $C_1$-$C_5$-Alkylthio-$C_2$-$C_5$-alkyl, $C_1$-$C_5$-Dialkylamino-$C_2$-$C_5$-alkyl, Phenyl-$C_1$-$C_5$-alkyl, $C_1$-$C_5$-Alkoxycarbonyl-$C_1$-$C_5$-alkyl, einen Rest der Formel $-(CH_2)_n-Y$, in der n für eine Zahl von 1 bis 5 und Y für Pyrrolidino, Piperidino, Morpholino, Thiomorpholino, Piperazino, N-($C_1$-$C_4$-Alkyl)piperazino oder Hexamethylenimino stehen, Phenyl, Pyridyl, Amino oder $C_1$-$C_5$-Dialkylamino,

$R^2$ Wasserstoff, $C_1$-$C_4$-Alkyl, Allyl, Benzyl oder Phenyl,

$R^3$ Wasserstoff, $C_1$-$C_4$-Alkyl, Allyl oder Benzyl oder $R^2$ und $R^3$ zusammen einen Rest der Formel

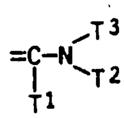 ,

in der $T^1$ für Wasserstoff oder $C_3$-$C_4$-Alkyl, $T^2$ für $C_1$-$C_4$-Alkyl oder Phenyl und $T^3$ für $C_1$-$C_4$-Alkyl stehen, oder $R^2$ und $R^3$ zusammen mit dem sie verbindenden Stickstoffatom Pyrrolidino, Piperidino, Morpholino, Thiomorpholino, Piperazino, N-($C_1$-$C_4$-Alkyl)piperazino oder Hexamethylenimino,

$R^4$ Cyano, Carbamoyl, Thiocarbamoyl oder den Rest der Formel

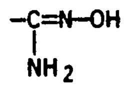 ,

$R^5$ Fluor, Chlor, Brom, Hydroxy, $C_1$-$C_6$-Alkanoyloxy oder Benzoyloxy und

$R^6$ Wasserstoff, $C_1$-$C_4$-Alkyl, Phenyl, $C_1$-$C_6$-Alkanoyl, Benzoyl, Cyano, Chlor, Brom, Nitro, Hydroxysulfonyl oder einen Rest der Formel

$-CH=NOH$ oder 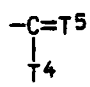

bedeuten, wobei $T^4$ und $T^5$ jeweils die obengenannte Bedeutung besitzen.

Besonders bevorzugt sind Pyrrolderivate der Formel I in der

$R^1$ Wasserstoff, $C_1$-$C_{10}$-Alkyl, $C_3$-$C_4$-Alkenyl, Benzyl, Phenyl oder Amino,

$R^2$ Wasserstoff, $C_1$-$C_4$-Alkyl, Allyl, Benzyl oder Phenyl,

$R^3$ Wasserstoff, $C_1$-$C_4$-Alkyl oder Allyl, oder $R^2$ und $R^3$ zusammen mit dem sie verbindenden Stickstoffatom Pyrrolidino, Piperidino, Morpholino, Thiomorpholino, N-Methylpiperazino oder Hexamethylenimino,

$R^4$ Cyano, Carbamoyl, Thiocarbamoyl oder den Rest der Formel

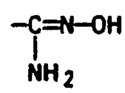 ,

$R^5$ Chlor, Brom, Hydroxy, $C_1$-$C_6$-Alkanoyloxy oder Benzoyloxy und

$R^6$ Wasserstoff, $C_1$-$C_4$-Alkyl, Phenyl, $C_1$-$C_6$-Alkanoyl, Benzoyl, Cyano oder einen Rest der Formel

$-CH=NOH$ , 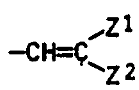 oder $CH=N-X^3$

bedeuten, wobei $Z^1$ und $Z^2$ gleich oder verschieden sind und unabhängig voneinander jeweils für Cyano, $C_1$-$C_4$-Alkoxycarbonyl oder Nitro stehen und $X^3$ die obengenannte Bedeutung besitzt.

Insbesondere hervorzuheben sind Pyrrolderivate der Formel I, in der

$R^1$ $C_1$-$C_4$-Alkyl, Phenyl oder Amino und

$R^2$ und $R^3$ jeweils Wasserstoff oder

$R^1$ Wasserstoff,

$R^2$ Methyl, Ethyl, Allyl, Benzyl oder Phenyl,

$R^3$ Methyl, Ethyl oder Allyl oder $R^2$ und $R^3$ zusammen mit dem sie verbindenden Stickstoffatom Pyrrolidino, Piperidino, Morpholino, N-Methylpiperazino oder Hexamethylenimino,

$R^4$ Cyano oder Carbamoyl,

$R^5$ Chlor, Brom oder Hydroxy,

$R^6$ Wasserstoff, Formyl, Cyano oder einen Rest der Formel

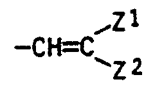

bedeuten, wobei $Z^1$ und $Z^2$ gleich oder verschieden sind und unabhängig voneinander jeweils für Cyano, $C_1$-$C_4$-Alkyl, $C_1$-$C_4$-Alkoxycarbonyl oder Nitro stehen.

Ganz besonders wichtige Pyrrolderivate sind in diesem Zusammenhang solche, wo die Reste $Z^1$ und $Z^2$ unabhängig voneinander Cyano oder $C_1$-$C_4$-Alkoxycarbonyl oder einer der beiden Reste auch Wasserstoff bedeuten.

Die folgenden Beispiele sollen die Erfindung näher erläutern.

## Beispiel 1

### 2-Amino-3-cyano-1-methylpyrrolin-4-on

99 g Malodinitril wurden in 150 ml N,N-Dimethylformamid (DMF) gelöst und auf 0 °C abgekühlt. Hierzu wurden unter Kühlung gleichzeitig 556 g Tributylamin und 169,5 g Chloracetylchlorid zugetropft und die Mischung wurde anschließend 10 Minuten bei 10 °C nachgerührt. Das Reaktionsgemisch wurde anschließend auf eine Mischung von 1,5 kg Eis und 327 ml 40 gew.%iger wässriger Methylaminlösung gegeben und 8 Stunden bei Raumtemperatur gerührt. Der Niederschlag wurde abgesaugt, nachgewaschen und bei 50 °C im Trockenschrank getrocknet. Man erhielt 124 g (60 % d. Th.) der Verbindung der Formel

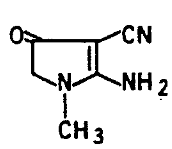

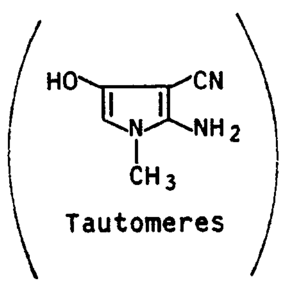


vom Schmelzpunkt 300 °C. IR-, NMR-, UV- und Massenspektren und die Elementaranalyse stehen mit der Konstitutionsformel im Einklang.

## Beispiel 2

### 2-Amino-3-cyano-1-ethyl-pyrrolin-4-on

19,8 g Malodinitril wurden in 30 ml DMF gelöst und auf 0 °C abgekühlt. Hierzu wurden unter Kühlung gleichzeitig 61 g Triethylamin und 32 g Chloracetylchlorid zugetropft und die Mischung 10 Minuten bei

10 °C gerührt. Anschließend wurden unter Kühlung 49 g 70 Gew.% wässrige Ethylaminlösung zugetropft und das Gemisch wurde 8 Stunden bei Raumtemperatur gerührt. Der Niederschlag wurde abgesaugt, nachgewaschen und bei 50 °C getrocknet. Man erhielt 41,8 g (92 %) verunreinigtes Rohprodukt, welches aus Isopropnaol/Wasser 1:1 unter Zusatz von Aktivkohle umkristallisiert wurde. Hieraus ergaben sich 10,9 g (24 %d. Th.) der Verbindung der Formel

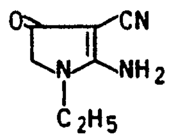

vom Schmelzpunkt 278 bis 280 °C. IR-, NMR-, UV- und Massenspektren und die Elementaranalyse stehen mit der Konstitutionsformel im Einklang.

Beispiel 3

2-Amino-3-cyano-1-isopropyl-pyrrolin-4-on

99 g Malodinitril wurden in 150 ml DMF gelöst und auf 0 °C abgekühlt. Hierzu wurden unter Kühlung gleichzeitig 306 g Triethylamin und 159 g Chloracetylchlorid zugetropft. Das Reaktionsgemisch wurde 10 Minuten bei 10 °C gerührt und anschließend auf eine Mischung von 1,5 kg Eis und 158 g Isopropylamin gegeben. Die Mischung wurde 8 Stunden bei Raumtemperatur gerührt. Anschließend wurden die flüchtigen Anteile bei 100 °C und Normaldruck abdestilliert und der Destillationsrückstand auf 5 °C abgekühlt. Der Niederschlag wurde abgesaugt, nachgewaschen und getrocknet. Man erhielt 105,6 g (43 % d. Th.) der Verbindung der Formel

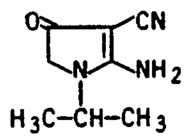

vom Schmelzpunkt 285 bis 290 °C. IR-, NMR-, UV- und Massenspektren und die Elementaranalyse stehen mit der Konstitutionsformel im Einklang.

Beispiel 4

2-Amino-3-cyano-1-tridecylpyrrolin-4-on

19,8 g Malodinitril wurden in 30 ml DMF gelöst und auf 0 °C abgekühlt. Hierzu wurden unter Kühlung gleichzeitig 61 g Triethylamin und 27 g Chloracetylchlorid zugetropft und das Gemisch 10 Minuten bei 10 °C gerührt Anschließend wurden 65,7 g Tridecylamin zugetropft und die Reaktionsmischung wurde 48 Stunden bei Raumtemperatur gerührt. Das Gemisch wurde danach mit 300 ml Isopropylamin versetzt, 2 Stunden unter Rückfluß gekocht, mit 10 g Aktivkohle versetzt und heiß abfiltriert. Nach dem Abkühlen wurde der Niederschlag abgesaugt, nachgewaschen und bei 50 °C getrocknet. Man erhielt 40 g (44 % d. Th.) der Verbindung der Formel

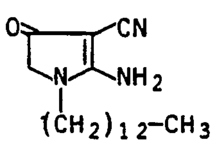

vom Schmelzpunkt 214 bis 215 °C. IR-, NMR-, UV- und Massenspektren und die Elementaranalyse stehen

mit der Konstitutionsformel im Einklang.

Beispiel 5

1-Allyl-2-amino-3-cyano-pyrrolin-4-on

19,8 g Malodinitril wurden in 30 ml DMF gelöst und auf 0 °C abgekühlt. Hierzu wurden unter Kühlung gleichzeitig 87 g Tributylamin und 27 g Chloracetylchlorid zugetropft und das Gemisch 10 Minuten bei 10 °C gerührt. Das Gemisch wurde anschließend auf eine Mischung von 300 g Eis und 43 g Allylamin gegeben und 8 Stunden bei Raumtemperatur gerührt. Der Niederschlag wurde abgesaugt, nachgewaschen und getrocknet. Man erhielt 38,1 g (78 % d. Th.) verunreinigtes Rohprodukt, welches zur Reinigung in 150 ml Isopropanol aufgekocht wurde. Nach dem Abkühlen auf 5 °C wurde der Niederschlag abgesaugt, nachgewaschen und getrocknet. Man erhielt 31,2 g (64 % d. Th.) der Verbindung der Formel

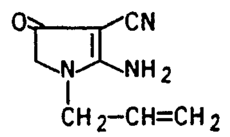

vom Schmelzpunkt 275 bis 278 °C. IR-, NMR-, UV- und Massenspektren und die Elementaranalyse stehen mit der Konstitutionsformel im Einklang.

Beispiel 6

2-Amino-3-cyano-1-(2-methoxyethyl)pyrrolin-4-on

99 g Malodinitril wurden in 150 ml DMF gelöst und auf 0 °C abgekühlt. Hierzu wurden unter Kühlung gleichzeitig 306 g Triethylamin und 159 g Chloracetylchlorid zugetropft und das Gemisch 10 Minuten bei 10 °C gerührt. Das Gemisch wurde anschließend auf eine Mischung von 115 kg Eis und 291 g 2-Methoxyethylamin gegeben und 8 Stunden bei Raumtemperatur gerührt. Die flüchtigen Anteile wurden bei 100 °C und Normaldruck abdestilliert und der Destillationsrückstand wurde am Rotationsverdampfer im Wasserstrahlvakuum eingeengt. Der Rückstand wurde aus 500 ml Isopropanol umkristallisiert. Der Niederschlag wurde abgesaugt, nachgewaschen und bei 50 °C getrocknet. Man erhielt 205,5 g (76 % d. Th.) der Verbindung der Formel

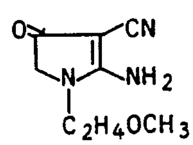

vom Schmelzpunkt 108 bis 110 °C. IR-, NMR-, UV- und Massenspektren und die Elementaranalyse stehen mit der Konstitutionsformel im Einklang.

Beispiel 7

2-Amino-3-cyano-1-phenylpyrrolin-4-on (Variante A)

26,4 g Malodinitril wurden in 150 ml DMF gelöst und auf 0 °C abgekühlt. Hierzu wurden unter Kühlung gleichzeitig 148 g Tributylamin und 45 g Chloracetylchlorid zugetropft und das Gemisch wurde 30 Minuten bei Raumtemperatur gerührt. Das Gemisch wurde anschließend mit 42 g Anilin versetzt und 15 Stunden bei 80 °C gerührt und anschließend mit 200 g Wasser versetzt. Nach dem Abkühlen auf Raumtemperatur

wurde die organische Phase abgetrennt und mit Dichlormethan digeriert. Der Niederschlag wurde abgesaugt, nachgewaschen und getrocknet. Man erhielt 37,4 g (46 % d. Th.) der Verbindung der Formel

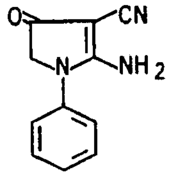

vom Schmelzpunkt 285 bis 290 °C. IR-, NMR-, UV- und Massenspektren und die Elementaranalyse stehen mit der Konstitutionsformel im Einklang.

(Variante B)

19,8 g Malodinitril wurden in 30 ml DMF gelöst und auf 0 °C abgekühlt. Hierzu wurden unter Kühlung gleichzeitig 61 g Triethylamin und 27 g Chloracetylchlorid zugetropft und das Gemisch wurde 10 Minuten bei 10 °C gerührt. Anschließend wurden 27,6 g Anilin zugesetzt und das Reaktionsgemisch wurde 8 Stunden bei Raumtemperatur gerührt. Nach Zugabe von 1 kg Wasser wurde 2 Stunden unter Rückfluß gekocht und der Niederschlag nach dem Abkühlen auf 10 °C abgesaugt, nachgewaschen und getrocknet. Man erhielt 39,2 g (66 % d. Th.) der Verbindung, welche mit dem nach Variante A) synthetisierten Produkt in allen Daten identisch ist.

In analoger Weise werden die in der folgenden Tabelle 1 aufgeführten Verbindungen erhalten.

Tabelle 1

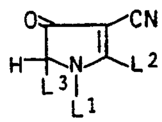

| Bsp.-Nr. | $L^1$ | $L^2$ | $L^3$ | Schmelzpunkt [°C] |
|---|---|---|---|---|
| 8 | $CH_2-CH(C_2H_5)-C_4H_9$ | $NH_2$ | H | |
| 9 | n-$C_3H_7$ | $NH_2$ | H | 257-267 |
| 10 | n-$C_4H_9$ | $NH_2$ | H | 182-192 |
| 11 | iso-$C_4H_9$ | $NH_2$ | H | |
| 12 | sec-$C_4H_9$ | $NH_2$ | H | |
| 13 | n-$C_5H_{11}$ | $NH_2$ | H | |
| 14 | Neo-$C_5H_{11}$ | $NH_2$ | H | |
| 15 | n-$C_6H_{13}$ | $NH_2$ | H | |
| 16 | n-$C_7H_{15}$ | $NH_2$ | H | |
| 17 | n-$C_8H_{17}$ | $NH_2$ | H | |
| 18 | n-$C_9H_{19}$ | $NH_2$ | H | |
| 19 | n-$C_{10}H_{21}$ | $NH_2$ | H | |
| 20 | n-$C_{11}H_{23}$ | $NH_2$ | H | |
| 21 | n-$C_{12}H_{25}$ | $NH_2$ | H | |
| 22 | n-$C_{14}H_{29}$ | $NH_2$ | H | |
| 23 | n-$C_{15}H_{31}$ | $NH_2$ | H | |
| 24 | n-$C_{16}H_{33}$ | $NH_2$ | H | |
| 25 | n-$C_{17}H_{35}$ | $NH_2$ | H | |
| 26 | n-$C_{18}H_{37}$ | $NH_2$ | H | |
| 27 | n-$C_{19}H_{39}$ | $NH_2$ | H | |
| 28 | n-$C_{20}H_{41}$ | $NH_2$ | H | |
| 29 | $CH_2C\equiv CH$ | $NH_2$ | H | |
| 30 | Cyclo-$C_3H_5$ | $NH_2$ | H | |
| 31 | Cyclo-$C_4H_7$ | $NH_2$ | H | |
| 32 | Cyclo-$C_5H_9$ | $NH_2$ | H | |
| 33 | Cyclo-$C_6H_{11}$ | $NH_2$ | H | 300 |
| 34 | Cyclo-$C_7H_{13}$ | $NH_2$ | H | |
| 35 | Cyclo-$C_8H_{15}$ | $NH_2$ | H | |
| 36 | Cyclo-$C_9H_{17}$ | $NH_2$ | H | |
| 37 | Cyclo-$C_{10}H_{19}$ | $NH_2$ | H | |
| 38 | $CH_2CH_2OH$ | $NH_2$ | H | 245-250 |
| 39 | $CH_2CH_2OC_2H_5$ | $NH_2$ | H | |
| 40 | $CH_2CH_2NH_2$ | $NH_2$ | H | |

Tabelle 1 - Fortsetzung

| Bsp.-Nr. | $L^1$ | $L^2$ | $L^3$ | Schmelzpunkt [°C] |
|---|---|---|---|---|
| 41 | $CH_2CH_2N(CH_3)_2$ | $NH_2$ | H | |
| 42 | $CH_2CH_2N(C_2H_5)_2$ | $NH_2$ | H | |
| 43 | $CH_2CH_2SCH_3$ | $NH_2$ | H | |
| 44 | $CH_2CH_2SC_2H_5$ | $NH_2$ | H | |
| 45 | $(CH_2)_3OH$ | $NH_2$ | H | |
| 46 | $(CH_2)_3OCH_3$ | $NH_2$ | H | |
| 47 | $(CH_2)_3OC_2H_5$ | $NH_2$ | H | |
| 48 | $(CH_2)_3NH_2$ | $NH_2$ | H | |
| 49 | $(CH_2)_3N(CH_3)_2$ | $NH_2$ | H | |
| 50 | $(CH_2)_3N(C_2H_5)_2$ | $NH_2$ | H | |
| 51 | $(CH_2)_3SCH_3$ | $NH_2$ | H | |
| 52 | $(CH_2)_3SC_2H_5$ | $NH_2$ | H | |
| 53 | $CH_2C_6H_5$ | $NH_2$ | H | 236-238 |
| 54 | $CH_2CH_2C_6H_5$ | $NH_2$ | H | 230-232 |
| 55 | $CH(CH_3)C_6H_5$ | $NH_2$ | H | |
| 56 | $CH(C_6H_5)_2$ | $NH_2$ | H | |
| 57 | $C_6H_4-(2)-OH$ | $NH_2$ | H | |
| 58 | $C_6H_4-(3)-OH$ | $NH_2$ | H | |
| 59 | $C_6H_4-(4)-OH$ | $NH_2$ | H | |
| 60 | $C_6H_4-(2)-OCH_3$ | $NH_2$ | H | |
| 61 | $C_6H_4-(3)-OCH_3$ | $NH_2$ | H | 234 |
| 62 | $C_6H_4-(4)-OCH_3$ | $NH_2$ | H | 270 |
| 63 | $C_6H_4-(2)-OC_2H_5$ | $NH_2$ | H | |
| 64 | $C_6H_4-(3)-OC_2H_5$ | $NH_2$ | H | |
| 65 | $C_6H_4-(4)-OC_2H_5$ | $NH_2$ | H | |
| 66 | $C_6H_4-(2)-Cl$ | $NH_2$ | H | |
| 67 | $C_6H_4-(3)-Cl$ | $NH_2$ | H | |
| 68 | $C_6H_4-(4)-Cl$ | $NH_2$ | H | |
| 69 | $C_6H_4-(2)-NO_2$ | $NH_2$ | H | |
| 70 | $C_6H_4-(3)-NO_2$ | $NH_2$ | H | |
| 71 | $C_6H_4-(4)-NO_2$ | $NH_2$ | H | |
| 72 | $C_6H_4-(2)-NH_2$ | $NH_2$ | H | |
| 73 | $C_6H_4-(3)-NH_2$ | $NH_2$ | H | |
| 74 | $C_6H_4-(4)-NH_2$ | $NH_2$ | H | |
| 75 | $C_6H_4-(2)-CH_3$ | $NH_2$ | H | |

Tabelle 1 - Fortsetzung

| Bsp.-Nr. | $L^1$ | $L^2$ | $L^3$ | Schmelzpunkt [°C] |
|---|---|---|---|---|
| 76 | $C_6H_4-(3)-CH_3$ | $NH_2$ | H | |
| 77 | $C_6H_4-(4)-CH_3$ | $NH_2$ | H | |
| 78 | $C_6H_4-(2)-C_2H_5$ | $NH_2$ | H | |
| 79 | $C_6H_4-(3)-C_2H_5$ | $NH_2$ | H | |
| 80 | $C_6H_4-(4)-C_2H_5$ | $NH_2$ | H | |
| 81 | $C_6H_4-(2)-CN$ | $NH_2$ | H | |
| 82 | $C_6H_4-(3)-CN$ | $NH_2$ | H | |
| 83 | $C_6H_4-(4)-CN$ | $NH_2$ | H | |
| 84 | $C_6H_4-(2)-COOCH_3$ | $NH_2$ | H | |
| 85 | $C_6H_4-(3)-COOCH_3$ | $NH_2$ | H | |
| 86 | $C_6H_4-(4)-COOCH_3$ | $NH_2$ | H | |
| 87 | Pyrid-2-yl | $NH_2$ | H | |
| 88 | Pyrid-3-yl | $NH_2$ | H | 300 |
| 89 | Pyrid-4-yl | $NH_2$ | H | |
| 90 | $(CH_2)_2-C_6H_3-$<br>$-(3)-Cl(2)-CH_3$ | $NH_2$ | H | 270 |
| 91 | $(CH_2)_2-C_6H_3-$<br>$-(3,4)-(OCH_3)_2$ | $NH_2$ | H | 220-225 |
| 92 | $(CH_2)_2-C_6H_4-$<br>$-(2)-Cl$ | $NH_2$ | H | über 270 |
| 93 | $C_6H_3-(3,4)-Cl_2$ | $NH_2$ | H | über 270 |
| 94 | $CH_2-COOCH_3$ | $NH_2$ | H | über 270 |
| 95 | $CH_2-COOC_2H_5$ | $NH_2$ | H | |
| 96 | $NH_2$ | $NH_2$ | H | 280-285 |
| 97 | $N(CH_3)_2$ | $NH_2$ | H | |
| 98 | $N(C_2H_5)_2$ | $NH_2$ | H | |
| 99 | H | $N(CH_3)_2$ | H | 228-235 |
| 100 | H | $N(C_2H_5)_2$ | H | 119-123 |
| 101 | H | $N(n-C_3H_7)_2$2 | H | |
| 102 | H | $N(n-C_4H_9)_2$2 | H | |
| 103 | H | 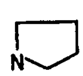 | H | 245-249 |
| 104 | H | 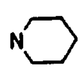 | H | 267-269 |
| 105 | H | 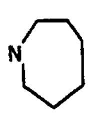 | H | |

Tabelle 1 - Fortsetzung

| Bsp.-Nr. | $L^1$ | $L^2$ | $L^3$ | Schmelzpunkt [°C] |
|---|---|---|---|---|
| 106 | H | 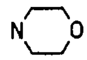 | H | 280-284 |
| 107 | H | 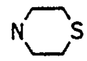 | H | |
| 108 | H | 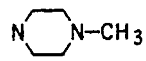 | H | |
| 109 | $CH_3$ | $NH_2$ | $CH_3$ | 298 |
| 110 | $C_2H_5$ | $NH_2$ | $CH_3$ | |
| 111 | iso-$C_3H_7$ | $NH_2$ | $CH_3$ | |
| 112 | $CH_2CH{=}CH_2$ | $NH_2$ | $CH_3$ | |
| 113 | Cyclo-$C_6H_{11}$ | $NH_2$ | $CH_3$ | |
| 114 | $CH_2CH_2OH$ | $NH_2$ | $CH_3$ | |
| 115 | $CH_2CH_2OCH_3$ | $NH_2$ | $CH_3$ | |
| 116 | $CH_2C_6H_5$ | $NH_2$ | $CH_3$ | |
| 117 | $C_6H_5$ | $NH_2$ | $CH_3$ | |
| 118 | $C_6H_4$-(4)-$OCH_3$ | $NH_2$ | $CH_3$ | |
| 119 | $C_6H_4$-(2)-$CH_3$ | $NH_2$ | $CH_3$ | |
| 120 | Pyrid-3-yl | $NH_2$ | $CH_3$ | |
| 121 | $NH_2$ | $NH_2$ | $CH_3$ | |
| 122 | H | $N(CH_3)_2$ | $CH_3$ | |
| 123 | H | $N(C_2H_5)_2$ | $CH_3$ | |
| 124 | H | 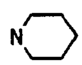 | $CH_3$ | |
| 125 | H | 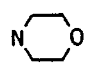 | $CH_3$ | |
| 126 | $CH_3$ | $NH_2$ | $C_6H_5$ | |
| 127 | $C_2H_5$ | $NH_2$ | $C_6H_5$ | |
| 128 | iso-$C_3H_7$ | $NH_2$ | $C_6H_5$ | |
| 129 | $CH_2CH{=}CH_2$ | $NH_2$ | $C_6H_5$ | |
| 130 | $CH_2CH_2OH$ | $NH_2$ | $C_6H_5$ | |
| 131 | $CH_2CH_2OCH_3$ | $NH_2$ | $C_6H_5$ | |
| 132 | $CH_2C_6H_5$ | $NH_2$ | $C_6H_5$ | |
| 133 | $C_6H_5$ | $NH_2$ | $C_6H_5$ | |
| 134 | $C_6H_4$-(4)-$OCH_3$ | $NH_2$ | $C_6H_5$ | |
| 135 | $C_6H_4$-(4)-CN | $NH_2$ | $C_6H_5$ | |

Tabelle 1 - Fortsetzung

| Bsp.-Nr. | $L^1$ | $L^2$ | $L^3$ | Schmelzpunkt [°C] |
|---|---|---|---|---|
| 136 | $NH_2$ | $NH_2$ | $C_6H_5$ | |
| 137 | H | $N(CH_3)_2$ | $C_6H_5$ | |
| 138 | H | $N(C_2H_5)_2$ | $C_6H_5$ | |
| 139 | H | 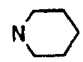 | $C_6H_5$ | |
| 140 | H | 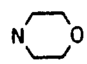 | $C_6H_5$ | |
| 141 | $(CH_2)_3-O-CH_2C_6H_5$ | $NH_2$ | H | 149 |
| 142 | $(CH_2)_3-O-(CH_2)_2-O-C_6H_5$ | $NH_2$ | H | 132 |

Beispiel 143

4-Chlor-3-cyano-2-dimethylformamidino-5-formyl-1-methyl-pyrrol

Zu 1,5 l DMF wurden bei 0 bis 5 °C 364 ml Phosphorylchlorid ($POCl_3$) getropft. Nach einer Stunde bei 0 bis 5 °C versetzte man die Reaktionsmischung tropfenweise mit einer Lösung von 137 g 2-Amino-3-cyano-1-methylpyrrolin-4-on (Beispiel 1) in 1,5 l DMF (exotherme Reaktion mit Temperaturanstieg auf 40 °C). Nach 4 Stunden Rühren bei 100 °C wurde auf Raumtemperatur abgekühlt, mit 5 l Wasser hydrolisiert und mit halbkonzentrierter Natronlauge auf pH 8 bis 9 eingestellt. Der beigefarbene Niederschlag wurde abgesaugt und im Trockenschrank bei 50 °C getrocknet. Man erhielt 180 g (76 % d. Th.) der Verbindung der Formel

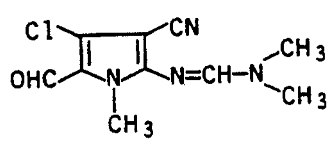

vom Schmelzpunkt 150 bis 152 °C. IR-, NMR-, UV- und Massenspektren und die Elementaranalyse stehen mit der Konstitutionsformel im Einklang.

Beispiel 144

4-Brom-3-cyano-2-dimethylformamidino-5-formyl-1-methylpyrrol

Zu 200 ml DMF wurden bei 0 bis 10 °C 172 g Phosphorylbromid ($POBr_3$) zugetropft. Das Gemisch wurde noch 5 Minuten nachgerührt und anschließend weitere 100 ml DMF zugegeben. In das Gemisch wurden 27,4 g 2-Amino-3-cyano-1-methylpyrrolin-4-on (Beispiel 1) portionsweise unter Kühlung eingetragen. Nach dem Abklingen der exothermen Reaktion wurde 2 Stunden bei 70 °C nachgerührt und die Reaktionslösung danach auf 2 kg Eiswasser gegossen. Die Suspension wurde bei Raumtemperatur 8 Stunden gerührt. Anschließend wurde der Niederschlag abgesaugt, nachgewaschen und bei 50 °C im Trockenschrank getrocknet. Man erhielt 12,6 g (22 %) der Verbindung der Formel

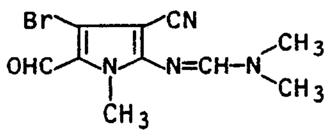

vom Schmelzpunkt 180 bis 184 °C. IR-, NMR-, UV- und Massenspektren und die Elementaranalyse stehen mit der Konstitutionsformel im Einklang.

In analoger Weise werden die in Tabelle 2 aufgeführten Verbindungen erhalten.

Tabelle 2

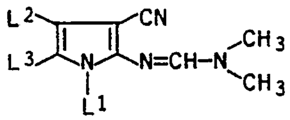

| Bsp.-Nr. | $L^1$ | $L^2$ | $L^3$ | Schmelzpunkt [°C] |
|---|---|---|---|---|
| 145 | $C_2H_5$ | Cl | CHO | |
| 146 | $n-C_3H_7$ | Cl | CHO | |
| 147 | $iso-C_3H_7$ | Cl | CHO | 136-139 |
| 148 | $n-C_4H_9$ | Cl | CHO | |
| 149 | $iso-C_4H_9$ | Cl | CHO | |
| 150 | $sec-C_4H_9$ | Cl | CHO | |
| 151 | $n-C_5H_{11}$ | Cl | CHO | |
| 152 | $Neo-C_5H_{11}$ | Cl | CHO | |
| 153 | $n-C_6H_{13}$ | Cl | CHO | |
| 154 | $n-C_7H_{15}$ | Cl | CHO | |
| 155 | $n-C_8H_{17}$ | Cl | CHO | |
| 156 | $n-C_9H_{19}$ | Cl | CHO | |
| 157 | $n-C_{10}H_{21}$ | Cl | CHO | |
| 158 | $n-C_{11}H_{23}$ | Cl | CHO | |
| 159 | $n-C_{12}H_{25}$ | Cl | CHO | |
| 160 | $n-C_{13}H_{27}$ | Cl | CHO | |
| 161 | $n-C_{14}H_{29}$ | Cl | CHO | |

Tabelle 2 - Fortsetzung

| Bsp.-Nr. | $L^1$ | $L^2$ | $L^3$ | Schmelzpunkt [°C] |
|---|---|---|---|---|
| 162 | n-$C_{15}H_{31}$ | Cl | CHO | |
| 163 | n-$C_{16}H_{33}$ | Cl | CHO | |
| 164 | n-$C_{17}H_{35}$ | Cl | CHO | |
| 165 | n-$C_{18}H_{37}$ | Cl | CHO | |
| 166 | n-$C_{19}H_{39}$ | Cl | CHO | |
| 167 | n-$C_{20}H_{41}$ | Cl | CHO | |
| 168 | Cyclo-$C_3H_5$ | Cl | CHO | |
| 169 | Cyclo-$C_4H_7$ | Cl | CHO | |
| 170 | Cyclo-$C_5H_9$ | Cl | CHO | |
| 171 | Cyclo-$C_6H_{11}$ | Cl | CHO | |
| 172 | Cyclo-$C_7H_{13}$ | Cl | CHO | |
| 173 | Cyclo-$C_8H_{15}$ | Cl | CHO | |
| 174 | Cyclo-$C_9H_{17}$ | Cl | CHO | |
| 175 | Cyclo-$C_{10}H_{19}$ | Cl | CHO | |
| 176 | $CH_2CH_2Cl$ | Cl | CHO | 119-123 |
| 177 | $CH_2CH_2OCH_3$ | Cl | CHO | |
| 178 | $CH_2CH_2OC_2H_5$ | Cl | CHO | |
| 179 | $CH_2CH_2N{=}CH{-}N(CH_3)_2$ | Cl | CHO | |
| 180 | $CH_2CH_2N(CH_3)_2$ | Cl | CHO | |
| 181 | $CH_2CH_2N(C_2H_5)_2$ | Cl | CHO | |
| 182 | $CH_2CH_2SCH_3$ | Cl | CHO | |
| 183 | $CH_2CH_2SC_2H_5$ | Cl | CHO | |
| 184 | $(CH_2)_3Cl$ | Cl | CHO | |
| 185 | $(CH_2)_3OCH_3$ | Cl | CHO | |
| 186 | $(CH_2)_3OC_2H_5$ | Cl | CHO | |
| 187 | $(CH_2)_3N{=}CH{-}N(CH_3)_2$ | Cl | CHO | |
| 188 | $(CH_2)_3N(CH_3)_2$ | Cl | CHO | |
| 189 | $(CH_2)_3N(C_2H_5)_2$ | Cl | CHO | |
| 190 | $(CH_2)_3SCH_3$ | Cl | CHO | |
| 191 | $(CH_2)_3SC_2H_5$ | Cl | CHO | |
| 192 | $CH_2C_6H_5$ | Cl | CHO | |
| 193 | $CH_2CH_2C_6H_5$ | Cl | CHO | |
| 194 | $CH(CH_3)C_6H_5$ | Cl | CHO | |
| 195 | $CH(C_6H_5)_2$ | Cl | CHO | |
| 196 | $C_6H_5$ | Cl | CHO | 175-176 |

Tabelle 2 - Fortsetzung

| Bsp.-Nr. | $L^1$ | $L^2$ | $L^3$ | Schmelzpunkt [°C] |
|---|---|---|---|---|
| 197 | $C_6H_4$-(2)-Cl | Cl | CHO | |
| 198 | $C_6H_4$-(3)-Cl | Cl | CHO | |
| 199 | $C_6H_4$-(4)-Cl | Cl | CHO | |
| 200 | $C_6H_4$-(2)-$OCH_3$ | Cl | CHO | |
| 201 | $C_6H_4$-(3)-$OCH_3$ | Cl | CHO | 123-130 |
| 202 | $C_6H_4$-(4)-$OCH_3$ | Cl | CHO | 140-146 |
| 203 | $C_6H_4$-(2)-$OC_2H_5$ | Cl | CHO | |
| 204 | $C_6H_4$-(3)-$OC_2H_5$ | Cl | CHO | |
| 205 | $C_6H_4$-(4)-$OC_2H_5$ | Cl | CHO | |
| 206 | $C_6H_4$-(2)-$NO_2$ | Cl | CHO | |
| 207 | $C_6H_4$-(3)-$NO_2$ | Cl | CHO | |
| 208 | $C_6H_4$-(4)-$NO_2$ | Cl | CHO | |
| 209 | $CH_2$-$COOCH_3$ | Cl | CHO | |
| 210 | $CH_2$-$COOC_2H_5$ | Cl | CHO | |
| 211 | N=CH-N$(CH_3)_2$ | Cl | CHO | 183 |
| 212 | $N(CH_3)_2$ | Cl | CHO | |
| 213 | $N(C_2H_5)_2$ | Cl | CHO | |
| 214 | $C_2H_5$ | Br | CHO | |
| 215 | n-$C_3H_7$ | Br | CHO | |
| 216 | iso-$C_3H_7$ | Br | CHO | |
| 217 | n-$C_4H_9$ | Br | CHO | |
| 218 | n-$C_{13}H_{27}$ | Br | CHO | |
| 219 | Cyclo-$C_6H_{11}$ | Br | CHO | |
| 220 | $C_6H_5$ | Br | CHO | |
| 221 | $C_2H_4Br$ | Br | CHO | |
| 222 | N=CH-N$(CH_3)_2$ | Br | CHO | |
| 223 | $N(CH_3)_2$ | Br | CHO | |
| 224 | $N(C_2H_5)_2$ | Br | CHO | |
| 225 | $CH_3$ | Cl | $CH_3$ | |
| 226 | $C_2H_5$ | Cl | $CH_3$ | |
| 227 | iso-$C_3H_7$ | Cl | $CH_3$ | |
| 228 | $CH_2CH$=$CH_2$ | Cl | $CH_3$ | |
| 229 | Cyclo-$C_6H_{11}$ | Cl | $CH_3$ | |
| 230 | $CH_2CH_2Cl$ | Cl | $CH_3$ | |
| 231 | $CH_2CH_2OCH_3$ | Cl | $CH_3$ | |

Tabelle 2 - Fortsetzung

| Bsp.-Nr. | $L^1$ | $L^2$ | $L^3$ | Schmelzpunkt [°C] |
|---|---|---|---|---|
| 232 | $CH_2C_6H_5$ | Cl | $CH_3$ | |
| 233 | $C_6H_5$ | Cl | $CH_3$ | |
| 234 | Pyrid-3-yl | Cl | $CH_3$ | |
| 235 | $CH_3$ | Br | $CH_3$ | |
| 236 | $C_2H_5$ | Br | $CH_3$ | |
| 237 | iso-$C_3H_7$ | Br | $CH_3$ | |
| 238 | $CH_2CH{=}CH_2$ | Br | $CH_3$ | |
| 239 | Cyclo-$C_6H_{11}$ | Br | $CH_3$ | |
| 240 | $CH_2CH_2Br$ | Br | $CH_3$ | |
| 241 | $CH_2CH_2OCH_3$ | Br | $CH_3$ | |
| 242 | $CH_2C_6H_5$ | Br | $CH_3$ | |
| 243 | $C_6H_5$ | Br | $CH_3$ | |
| 244 | Pyrid-3-yl | Br | $CH_3$ | |
| 245 | $CH_3$ | Cl | $C_6H_5$ | |
| 246 | $C_2H_5$ | Cl | $C_6H_5$ | |
| 247 | iso-$C_3H_7$ | Cl | $C_6H_5$ | |
| 248 | $CH_2CH{=}CH_2$ | Cl | $C_6H_5$ | |
| 249 | Cyclo-$C_6H_{11}$ | Cl | $C_6H_5$ | |
| 250 | $CH_2CH_2Cl$ | Cl | $C_6H_5$ | |
| 251 | $CH_2CH_2OCH_3$ | Cl | $C_6H_5$ | |
| 252 | $CH_2C_6H_5$ | Cl | $C_6H_5$ | |
| 253 | $C_6H_5$ | Cl | $C_6H_5$ | |
| 254 | Pyrid-3-yl | Cl | $C_6H_5$ | |
| 255 | $CH_3$ | Br | $C_6H_5$ | |
| 256 | $C_2H_5$ | Br | $C_6H_5$ | |
| 257 | iso-$C_3H_7$ | Br | $C_6H_5$ | |
| 258 | $CH_2CH{=}CH_2$ | Br | $C_6H_5$ | |
| 259 | Cyclo-$C_6H_{11}$ | Br | $C_6H_5$ | |
| 260 | $CH_2CH_2Br$ | Br | $C_6H_5$ | |
| 261 | $CH_2CH_2OCH_3$ | Br | $C_6H_5$ | |
| 262 | $CH_2C_6H_5$ | Br | $C_6H_5$ | |
| 263 | $C_6H_5$ | Br | $C_6H_5$ | |
| 264 | Pyrid-3-yl | Br | $C_6H_5$ | |

Beispiel 265

4-Chlor-3-cyano-2-dimethylformamidino-1-methylpyrrol

Zu 285 ml DMF wurden bei 0 bis 5 °C 33 ml Phosphorylchlorid ($POCl_3$) getropft. Nach einer Stunde bei 0 bis 5 °C trug man 41,1 g 2-Amino-3-cyano-1-methylpyrrolin-4-on (Beispiel 1) portionsweise in die Reaktionslösung (exotherme Reaktion mit Temperaturanstieg auf 40 °C) ein. Nach 1 Stunde Rühren bei 70 °C wurde auf Raumtemperatur abgekühlt, mit 1,5 l Wasser hydrolisiert und mit 240 g Natriumacetat auf pH 7 eingestellt. Der Niederschlag wurde abgesaugt und im Trockenschrank bei 50 °C getrocknet. Man erhielt 40,5 g (75 % d. Th.) der Verbindung der Formel

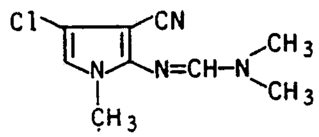

vom Schmelzpunkt 88 °C. IR-, NMR-, UV- und Massenspektren und die Elementaranalyse stehen mit der Konstitutionsformel im Einklang.

Beispiel 266

4-Brom-3-cyano-2-dimethylformamidino-1-methylpyrrol

Zu 190 ml DMF wurden bei 0 bis 10 °C 70 g Phosphorylbromid ($POBr_3$) getropft. Nach einer Stunde bei 0 bis 5 °C trug man 27 g 2-Amino-3-cyano-1-methylpyrrolin-4-on (Beispiel 1) portionsweise in die Reaktionslösung (exotherme Reaktion mit Temperaturanstieg auf 30 °C) ein. Nach 1 Stunde Rühren bei 80 °C wurde auf Raumtemperatur abgekühlt, mit 1,5 l Wasser hydrolysiert und mit 160 g Natriumacetat auf pH 7 eingestellt. Der Niederschlag wurde abgesaugt und im Trockenschrank bei 50 °C getrocknet. Man erhielt 22,5 g (44 % d. Th.) der Verbindung der Formel

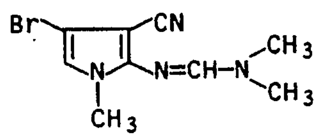 .

IR-, NMR-, UV- und Massenspektren und die Elementaranalyse stehen mit der Konstitutionsformel im Einklang.

In analoger Weise werden die in der folgenden Tabelle 3 aufgeführten Verbindungen erhalten.

Tabelle 3

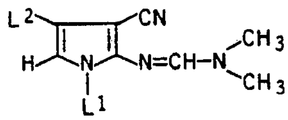

| Bsp.-Nr. | $L^1$ | $L^2$ | Schmelzpunkt [°C] |
|---|---|---|---|
| 267 | $C_2H_5$ | Cl | |
| 268 | n-$C_3H_7$ | Cl | |
| 269 | iso-$C_3H_7$ | Cl | |
| 270 | n-$C_4H_9$ | Cl | |
| 271 | iso-$C_4H_9$ | Cl | |
| 272 | sec-$C_4H_9$ | Cl | |
| 273 | n-$C_5H_{11}$ | Cl | |
| 274 | Neo-$C_5H_{11}$ | Cl | |
| 275 | n-$C_6H_{13}$ | Cl | |
| 276 | n-$C_7H_{15}$ | Cl | |
| 277 | n-$C_8H_{17}$ | Cl | |
| 278 | n-$C_9H_{19}$ | Cl | |
| 279 | n-$C_{10}H_{21}$ | Cl | |
| 280 | n-$C_{11}H_{23}$ | Cl | |
| 281 | n-$C_{12}H_{25}$ | Cl | |
| 282 | n-$C_{13}H_{27}$ | Cl | |
| 283 | n-$C_{14}H_{29}$ | Cl | |
| 284 | n-$C_{15}H_{31}$ | Cl | |
| 285 | n-$C_{16}H_{33}$ | Cl | |
| 286 | n-$C_{17}H_{35}$ | Cl | |
| 287 | n-$C_{18}H_{37}$ | Cl | |
| 288 | n-$C_{19}H_{39}$ | Cl | |
| 289 | n-$C_{20}H_{41}$ | Cl | |
| 290 | Cyclo-$C_3H_5$ | Cl | |
| 291 | Cyclo-$C_4H_7$ | Cl | |
| 292 | Cyclo-$C_5H_9$ | Cl | |
| 293 | Cyclo-$C_6H_{11}$ | Cl | |
| 294 | Cyclo-$C_7H_{13}$ | Cl | |
| 295 | Cyclo-$C_8H_{15}$ | Cl | |
| 296 | Cyclo-$C_9H_{17}$ | Cl | |
| 297 | Cyclo-$C_{10}H_{19}$ | Cl | |
| 298 | $CH_2CH_2Cl$ | Cl | |

Tabelle 3 - Fortsetzung

| Bsp.-Nr. | $L^1$ | $L^2$ | Schmelzpunkt [°C] |
|---|---|---|---|
| 299 | $CH_2CH_2OCH_3$ | Cl | |
| 300 | $CH_2CH_2OC_2H_5$ | Cl | |
| 301 | $CH_2CH_2N{=}CH{-}N(CH_3)_2$ | Cl | |
| 302 | $CH_2CH_2N(CH_3)_2$ | Cl | |
| 303 | $CH_2CH_2N(C_2H_5)_2$ | Cl | |
| 304 | $CH_2CH_2SCH_3$ | Cl | |
| 305 | $CH_2CH_2SC_2H_5$ | Cl | |
| 306 | $(CH_2)_3Cl$ | Cl | |
| 307 | $(CH_2)_3OCH_3$ | Cl | |
| 308 | $(CH_2)_3OC_2H_5$ | Cl | |
| 309 | $(CH_2)_3N{=}CH{-}N(CH_3)_2$ | Cl | |
| 310 | $(CH_2)_3N(CH_3)_2$ | Cl | |
| 311 | $(CH_2)_3N(C_2H_5)_2$ | Cl | |
| 312 | $(CH_2)_3SCH_3$ | Cl | |
| 313 | $(CH_2)_3SC_2H_5$ | Cl | |
| 314 | $CH_2C_6H_5$ | Cl | |
| 315 | $CH_2CH_2C_6H_5$ | Cl | |
| 316 | $CH(CH_3)C_6H_5$ | Cl | |
| 317 | $CH(C_6H_5)_2$ | Cl | |
| 318 | $C_6H_5$ | Cl | |
| 319 | $C_6H_4$-(2)-Cl | Cl | |
| 320 | $C_6H_4$-(3)-Cl | Cl | |
| 321 | $C_6H_4$-(4)-Cl | Cl | |
| 322 | $C_6H_4$-(2)-$OCH_3$ | Cl | |
| 323 | $C_6H_4$-(3)-$OCH_3$ | Cl | |
| 324 | $C_6H_4$-(4)-$OCH_3$ | Cl | |
| 325 | $C_6H_4$-(2)-$OC_2H_5$ | Cl | |
| 326 | $C_6H_4$-(3)-$OC_2H_5$ | Cl | |
| 327 | $C_6H_4$-(4)-$OC_2H_5$ | Cl | |
| 328 | $C_6H_4$-(2)-$NO_2$ | Cl | |
| 329 | $C_6H_4$-(3)-$NO_2$ | Cl | |
| 330 | $C_6H_4$-(4)-$NO_2$ | Cl | |

Tabelle 3 - Fortsetzung

| Bsp.-Nr. | $L^1$ | $L^2$ | Schmelzpunkt [°C] |
|---|---|---|---|
| 331 | $CH_2-COOCH_3$ | Cl | |
| 332 | $CH_2-COOC_2H_5$ | Cl | |
| 333 | $N=CH-N(CH_3)_2$ | Cl | |
| 334 | $N(CH_3)_2$ | Cl | |
| 335 | $N(C_2H_5)_2$ | Cl | |
| 336 | $C_2H_5$ | Br | |
| 337 | iso-$C_3H_7$ | Br | |
| 338 | Cyclo-$C_6H_{11}$ | Br | |
| 339 | $CH_2CH_2Br$ | Br | |
| 340 | $CH_2C_6H_5$ | Br | |
| 341 | $C_6H_5$ | Br | |
| 342 | $N=CH-N(CH_3)_2$ | Br | |
| 343 | $N(CH_3)_2$ | Br | |
| 344 | $N(C_2H_5)_2$ | Br | |

Beispiel 345

2-Amino-3-chlor-4-cyano-5-formyl-1-methylpyrrol

84,8 g 3-Chlor-4-cyano-5-formyl-1-methyl-2-dimethylaminoformamido-pyrrol (Beispiel 143) wurden in 50 ml Wasser und 150 ml Ethanol gelöst. Hierzu wurde eine Lösung von 15 g KOH in 150 ml Ethanol gegeben und das Gemisch 10 Minuten unter Rückfluß gekocht. Anschließend wurde auf 1 kg Eiswasser gegossen, mit konz. Ameisensäure auf pH 7 eingestellt, der Niederschlag abgesaugt, nachgewaschen und bei 50 °C im Trockenschrank getrocknet. Man erhielt 60,2 g (92 % d. Th.) der Verbindung der Formel

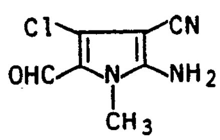

vom Schmelzpunkt 282 bis 285 °C. IR-, NMR-, UV- und Massenspektren und die Elementaranalyse stehen mit der Konstitutionsformel im Einklang.

Analog Beispiel 345 werden die in Tabelle 4 aufgeführten Verbindungen erhalten.

Tabelle 4

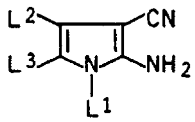

| Bsp.-Nr. | $L^1$ | $L^2$ | $L^3$ | Schmelzpunkt [°C] |
|---|---|---|---|---|
| 346 | $C_2H_5$ | Cl | CHO | |
| 347 | n-$C_3H_7$ | Cl | CHO | |
| 348 | iso-$C_3H_7$ | Cl | CHO | 180-195 |
| 349 | n-$C_4H_9$ | Cl | CHO | |
| 350 | iso-$C_4H_9$ | Cl | CHO | |
| 351 | sec-$C_4H_9$ | Cl | CHO | |
| 352 | n-$C_5H_{11}$ | Cl | CHO | |
| 353 | Neo-$C_5H_{11}$ | Cl | CHO | |
| 354 | n-$C_6H_{13}$ | Cl | CHO | |
| 355 | n-$C_7H_{15}$ | Cl | CHO | |
| 356 | n-$C_8H_{17}$ | Cl | CHO | |
| 357 | n-$C_9H_{19}$ | Cl | CHO | |
| 358 | n-$C_{10}H_{21}$ | Cl | CHO | |
| 359 | n-$C_{11}H_{23}$ | Cl | CHO | |
| 360 | n-$C_{12}H_{25}$ | Cl | CHO | |
| 361 | n-$C_{13}H_{27}$ | Cl | CHO | |
| 362 | n-$C_{14}H_{29}$ | Cl | CHO | |
| 363 | n-$C_{15}H_{31}$ | Cl | CHO | |
| 364 | n-$C_{16}H_{33}$ | Cl | CHO | |
| 365 | n-$C_{17}H_{35}$ | Cl | CHO | |
| 366 | n-$C_{18}H_{37}$ | Cl | CHO | |
| 367 | n-$C_{19}H_{39}$ | Cl | CHO | |
| 368 | n-$C_{20}H_{41}$ | Cl | CHO | |
| 369 | Cyclo-$C_3H_5$ | Cl | CHO | |
| 370 | Cyclo-$C_4H_7$ | Cl | CHO | |
| 371 | Cyclo-$C_5H_9$ | Cl | CHO | |
| 372 | Cyclo-$C_6H_{11}$ | Cl | CHO | |
| 373 | Cyclo-$C_7H_{13}$ | Cl | CHO | |
| 374 | Cyclo-$C_8H_{15}$ | Cl | CHO | |
| 375 | Cyclo-$C_9H_{17}$ | Cl | CHO | |
| 376 | Cyclo-$C_{10}H_{19}$ | Cl | CHO | |
| 377 | $CH_2CH_2Cl$ | Cl | CHO | |

Tabelle 4 - Fortsetzung

| Bsp.-Nr. | $L^1$ | $L^2$ | $L^3$ | Schmelzpunkt [°C] |
|---|---|---|---|---|
| 378 | $CH_2CH_2OCH_3$ | Cl | CHO | |
| 379 | $CH_2CH_2OC_2H_5$ | Cl | CHO | |
| 380 | $CH_2CH_2NH_2$ | Cl | CHO | |
| 381 | $CH_2CH_2N(CH_3)_2$ | Cl | CHO | |
| 382 | $CH_2CH_2N(C_2H_5)_2$ | Cl | CHO | |
| 383 | $CH_2CH_2SCH_3$ | Cl | CHO | |
| 384 | $CH_2CH_2SC_2H_5$ | Cl | CHO | |
| 385 | $(CH_2)_3Cl$ | Cl | CHO | |
| 386 | $(CH_2)_3OCH_3$ | Cl | CHO | |
| 387 | $(CH_2)_3OC_2H_5$ | Cl | CHO | |
| 388 | $(CH_2)_3NH_2$ | Cl | CHO | |
| 389 | $(CH_2)_3N(CH_3)_2$ | Cl | CHO | |
| 390 | $(CH_2)_3N(C_2H_5)_2$ | Cl | CHO | |
| 391 | $(CH_2)_3SCH_3$ | Cl | CHO | |
| 392 | $(CH_2)_3SC_2H_5$ | Cl | CHO | |
| 393 | $CH_2C_6H_5$ | Cl | CHO | |
| 394 | $CH_2CH_2C_6H_5$ | Cl | CHO | |
| 395 | $CH(CH_3)C_6H_5$ | Cl | CHO | |
| 396 | $CH(C_6H_5)_2$ | Cl | CHO | |
| 397 | $C_6H_5$ | Cl | CHO | 220-226 |
| 398 | $C_6H_4$-(2)-Cl | Cl | CHO | |
| 399 | $C_6H_4$-(3)-Cl | Cl | CHO | |
| 400 | $C_6H_4$-(4)-Cl | Cl | CHO | |
| 401 | $C_6H_4$-(2)-$OCH_3$ | Cl | CHO | |
| 402 | $C_6H_4$-(3)-$OCH_3$ | Cl | CHO | 123-130 |
| 403 | $C_6H_4$-(4)-$OCH_3$ | Cl | CHO | 140-146 |
| 404 | $C_6H_4$-(2)-$OC_2H_5$ | Cl | CHO | |
| 405 | $C_6H_4$-(3)-$OC_2H_5$ | Cl | CHO | |
| 406 | $C_6H_4$-(4)-$OC_2H_5$ | Cl | CHO | |
| 407 | $C_6H_4$-(2)-$NO_2$ | Cl | CHO | |
| 408 | $C_6H_4$-(3)-$NO_2$ | Cl | CHO | |
| 409 | $C_6H_4$-(4)-$NO_2$ | Cl | CHO | |

Tabelle 4 - Fortsetzung

| Bsp.-Nr. | $L^1$ | $L^2$ | $L^3$ | Schmelzpunkt [°C] |
|---|---|---|---|---|
| 410 | $CH_2$–$COOCH_3$ | Cl | CHO | |
| 411 | $CH_2$–$COOC_2H_5$ | Cl | CHO | |
| 412 | $NH_2$ | Cl | CHO | |
| 413 | $N(CH_3)_2$ | Cl | CHO | |
| 414 | $N(C_2H_5)_2$ | Cl | CHO | |
| 415 | $CH_3$ | Br | CHO | |
| 416 | $C_2H_5$ | Br | CHO | |
| 417 | n-$C_3H_7$ | Br | CHO | |
| 418 | iso-$C_3H_7$ | Br | CHO | |
| 419 | n-$C_4H_9$ | Br | CHO | |
| 420 | n-$C_{13}H_{17}$ | Br | CHO | |
| 421 | Cyclo-$C_6H_{11}$ | Br | CHO | |
| 422 | $C_6H_5$ | Br | CHO | |
| 423 | $C_2H_4Br$ | Br | CHO | |
| 424 | $NH_2$ | Br | CHO | |
| 425 | $N(CH_3)_2$ | Br | CHO | |
| 426 | $N(C_2H_5)_2$ | Br | CHO | |
| 427 | $CH_3$ | Cl | $CH_3$ | |
| 428 | $C_2H_5$ | Cl | $CH_3$ | |
| 429 | n-$C_3H_7$ | Cl | $CH_3$ | |
| 430 | iso-$C_3H_7$ | Cl | $CH_3$ | |
| 431 | n-$C_4H_9$ | Cl | $CH_3$ | |
| 432 | n-$C_{13}H_{17}$ | Cl | $CH_3$ | |
| 433 | Cyclo-$C_6H_{11}$ | Cl | $CH_3$ | |
| 434 | $C_6H_5$ | Cl | $CH_3$ | |
| 435 | $C_2H_4Br$ | Cl | $CH_3$ | |
| 436 | $NH_2$ | Cl | $CH_3$ | |
| 437 | $N(CH_3)_2$ | Cl | $CH_3$ | |
| 438 | $N(C_2H_5)_2$ | Cl | $CH_3$ | |
| 439 | $CH_3$ | Br | $CH_3$ | |
| 440 | $C_2H_5$ | Br | $CH_3$ | |
| 441 | n-$C_3H_7$ | Br | $CH_3$ | |

Tabelle 4 - Fortsetzung

| Bsp.-Nr. | $L^1$ | $L^2$ | $L^3$ | Schmelzpunkt [°C] |
|---|---|---|---|---|
| 442 | iso-$C_3H_7$ | Br | $CH_3$ | |
| 443 | n-$C_4H_9$ | Br | $CH_3$ | |
| 444 | n-$C_{13}H_{17}$ | Br | $CH_3$ | |
| 445 | Cyclo-$C_6H_{11}$ | Br | $CH_3$ | |
| 446 | $C_6H_5$ | Br | $CH_3$ | |
| 447 | $C_2H_4Br$ | Br | $CH_3$ | |
| 448 | $NH_2$ | Br | $CH_3$ | |
| 449 | $N(CH_3)_2$ | Br | $CH_3$ | |
| 450 | $N(C_2H_5)_2$ | Br | $CH_3$ | |
| 451 | $CH_3$ | Cl | $C_6H_5$ | |
| 452 | $C_2H_5$ | Cl | $C_6H_5$ | |
| 453 | n-$C_3H_7$ | Cl | $C_6H_5$ | |
| 454 | iso-$C_3H_7$ | Cl | $C_6H_5$ | |
| 455 | n-$C_4H_9$ | Cl | $C_6H_5$ | |
| 456 | n-$C_{13}H_{17}$ | Cl | $C_6H_5$ | |
| 457 | Cyclo-$C_6H_{11}$ | Cl | $C_6H_5$ | |
| 458 | $C_6H_5$ | Cl | $C_6H_5$ | |
| 459 | $C_2H_4Br$ | Cl | $C_6H_5$ | |
| 460 | $NH_2$ | Cl | $C_6H_5$ | |
| 461 | $N(CH_3)_2$ | Cl | $C_6H_5$ | |
| 462 | $N(C_2H_5)_2$ | Cl | $C_6H_5$ | |
| 463 | $CH_3$ | Br | $C_6H_5$ | |
| 464 | $C_2H_5$ | Br | $C_6H_5$ | |
| 465 | n-$C_3H_7$ | Br | $C_6H_5$ | |
| 466 | iso-$C_3H_7$ | Br | $C_6H_5$ | |
| 467 | n-$C_4H_9$ | Br | $C_6H_5$ | |
| 468 | n-$C_{13}H_{17}$ | Br | $C_6H_5$ | |
| 469 | Cyclo-$C_6H_{11}$ | Br | $C_6H_5$ | |
| 470 | $C_6H_5$ | Br | $C_6H_5$ | |
| 471 | $C_2H_4Br$ | Br | $C_6H_5$ | |
| 472 | $NH_2$ | Br | $C_6H_5$ | |
| 473 | $N(CH_3)_2$ | Br | $C_6H_5$ | |

Tabelle 4 - Fortsetzung

| Bsp.-Nr. | L1 | L2 | L3 | Schmelzpunkt [°C] |
|---|---|---|---|---|
| 474 | $N(C_2H_5)_2$ | Br | $C_6H_5$ | |
| 475 | $CH_3$ | Cl | H | |
| 476 | $C_2H_5$ | Cl | H | |
| 477 | n-$C_3H_7$ | Cl | H | |
| 478 | iso-$C_3H_7$ | Cl | H | |
| 479 | n-$C_4H_9$ | Cl | H | |
| 480 | n-$C_{13}H_{17}$ | Cl | H | |
| 481 | Cyclo-$C_6H_{11}$ | Cl | H | |
| 482 | $C_6H_5$ | Cl | H | |
| 483 | $C_2H_4Br$ | Cl | H | |
| 484 | $NH_2$ | Cl | H | |
| 485 | $N(CH_3)_2$ | Cl | H | |
| 486 | $N(C_2H_5)_2$ | Cl | H | |
| 487 | $CH_3$ | Br | H | |
| 488 | $C_2H_5$ | Br | H | |
| 489 | n-$C_3H_7$ | Br | H | |
| 490 | iso-$C_3H_7$ | Br | H | |
| 491 | n-$C_4H_9$ | Br | H | |
| 492 | n-$C_{13}H_{17}$ | Br | H | |
| 493 | Cyclo-$C_6H_{11}$ | Br | H | |
| 494 | $C_6H_5$ | Br | H | |
| 495 | $C_2H_4Br$ | Br | H | |
| 496 | $NH_2$ | Br | H | |
| 497 | $N(CH_3)_2$ | Br | H | |
| 498 | $N(C_2H_5)_2$ | Br | H | |

Beispiel 499

2-Amino-4-chlor-5-formyl-1-methylpyrrol-3-carbonsäureamid

9,54 g 4-Chlor-3-cyano-2-dimethylaminoformamidino-5-formyl-1-methylpyrrol (Beispiel 143) wurden in 100 ml 5 gew.%ger wässriger Natronhydroxidlösung eine Stunde unter Rückfluß erhitzt. Nach dem Abkühlen wurde der Niederschlag abgesaugt, nachgewaschen und getrocknet. Man erhielt 4,00 g (50 % d. Th.) der Verbindung der Formel

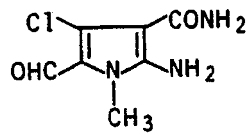

vom Schmelzpunkt 217 °C (Zersetzung). IR-, NMR-, UV- und Massenspektren und die Elementaranalyse stehen mit der Konstitutionsformel im Einklang.

Analog Beispiel 499 werden die in Tabelle 5 aufgeführten Verbindungen erhalten.

Tabelle 5

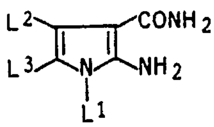

| Bsp.-Nr. | $L^1$ | $L^2$ | $L^3$ | Schmelzpunkt [°C] |
|---|---|---|---|---|
| 500 | $C_2H_5$ | Cl | CHO | |
| 501 | $n-C_3H_7$ | Cl | CHO | |
| 502 | $iso-C_3H_7$ | Cl | CHO | |
| 503 | $n-C_4H_9$ | Cl | CHO | |
| 504 | $iso-C_4H_9$ | Cl | CHO | |
| 505 | $sec-C_4H_9$ | Cl | CHO | |
| 506 | $n-C_5H_{11}$ | Cl | CHO | |
| 507 | $Neo-C_5H_{11}$ | Cl | CHO | |
| 508 | $n-C_6H_{13}$ | Cl | CHO | |
| 509 | $n-C_7H_{15}$ | Cl | CHO | |
| 510 | $n-C_8H_{17}$ | Cl | CHO | |
| 511 | $n-C_9H_{19}$ | Cl | CHO | |
| 512 | $n-C_{10}H_{21}$ | Cl | CHO | |
| 513 | $n-C_{11}H_{23}$ | Cl | CHO | |
| 514 | $n-C_{12}H_{25}$ | Cl | CHO | |
| 515 | $n-C_{13}H_{27}$ | Cl | CHO | |
| 516 | $n-C_{14}H_{29}$ | Cl | CHO | |

Tabelle 5 - Fortsetzung

| Bsp.-Nr. | $L^1$ | $L^2$ | $L^3$ | Schmelzpunkt [°C] |
|---|---|---|---|---|
| 517 | $n-C_{15}H_{31}$ | Cl | CHO | |
| 518 | $n-C_{16}H_{33}$ | Cl | CHO | |
| 519 | $n-C_{17}H_{35}$ | Cl | CHO | |
| 520 | $n-C_{18}H_{37}$ | Cl | CHO | |
| 521 | $n-C_{19}H_{39}$ | Cl | CHO | |
| 522 | $n-C_{20}H_{41}$ | Cl | CHO | |
| 523 | Cyclo-$C_3H_5$ | Cl | CHO | |
| 524 | Cyclo-$C_4H_7$ | Cl | CHO | |
| 525 | Cyclo-$C_5H_9$ | Cl | CHO | |
| 526 | Cyclo-$C_6H_{11}$ | Cl | CHO | |
| 527 | Cyclo-$C_7H_{13}$ | Cl | CHO | |
| 528 | Cyclo-$C_8H_{15}$ | Cl | CHO | |
| 529 | Cyclo-$C_9H_{17}$ | Cl | CHO | |
| 530 | Cyclo-$C_{10}H_{19}$ | Cl | CHO | |
| 531 | $CH_2CH_2Cl$ | Cl | CHO | |
| 532 | $CH_2CH_2OCH_3$ | Cl | CHO | |
| 533 | $CH_2CH_2OC_2H_5$ | Cl | CHO | |
| 534 | $CH_2CH_2NH_2$ | Cl | CHO | |
| 535 | $CH_2CH_2N(CH_3)_2$ | Cl | CHO | |
| 536 | $CH_2CH_2N(C_2H_5)_2$ | Cl | CHO | |
| 537 | $CH_2CH_2SCH_3$ | Cl | CHO | |
| 538 | $CH_2CH_2SC_2H_5$ | Cl | CHO | |
| 539 | $(CH_2)_3Cl$ | Cl | CHO | |
| 540 | $(CH_2)_3OCH_3$ | Cl | CHO | |
| 541 | $(CH_2)_3OC_2H_5$ | Cl | CHO | |
| 542 | $(CH_2)_3NH_2$ | Cl | CHO | |
| 543 | $(CH_2)_3N(CH_3)_2$ | Cl | CHO | |
| 544 | $(CH_2)_3N(C_2H_5)_2$ | Cl | CHO | |
| 545 | $(CH_2)_3SCH_3$ | Cl | CHO | |
| 546 | $(CH_2)_3SC_2H_5$ | Cl | CHO | |
| 547 | $CH_2C_6H_5$ | Cl | CHO | |
| 548 | $CH_2CH_2C_6H_5$ | Cl | CHO | |

Tabelle 5 - Fortsetzung

| Bsp.-Nr. | $L^1$ | $L^2$ | $L^3$ | Schmelzpunkt [°C] |
|---|---|---|---|---|
| 549 | $CH(CH_3)C_6H_5$ | Cl | CHO | |
| 550 | $CH(C_6H_5)_2$ | Cl | CHO | |
| 551 | $C_6H_5$ | Cl | CHO | |
| 552 | $C_6H_4$-(2)-Cl | Cl | CHO | |
| 553 | $C_6H_4$-(3)-Cl | Cl | CHO | |
| 554 | $C_6H_4$-(4)-Cl | Cl | CHO | |
| 555 | $C_6H_4$-(2)-$OCH_3$ | Cl | CHO | |
| 556 | $C_6H_4$-(3)-$OCH_3$ | Cl | CHO | |
| 557 | $C_6H_4$-(4)-$OCH_3$ | Cl | CHO | |
| 558 | $C_6H_4$-(2)-$OC_2H_5$ | Cl | CHO | |
| 559 | $C_6H_4$-(3)-$OC_2H_5$ | Cl | CHO | |
| 560 | $C_6H_4$-(4)-$OC_2H_5$ | Cl | CHO | |
| 561 | $C_6H_4$-(2)-$NO_2$ | Cl | CHO | |
| 562 | $C_6H_4$-(3)-$NO_2$ | Cl | CHO | |
| 563 | $C_6H_4$-(4)-$NO_2$ | Cl | CHO | |
| 564 | $CH_2$-$COOCH_3$ | Cl | CHO | |
| 565 | $CH_2$-$COOC_2H_5$ | Cl | CHO | |
| 566 | $NH_2$ | Cl | CHO | |
| 567 | $N(CH_3)_2$ | Cl | CHO | |
| 568 | $N(C_2H_5)_2$ | Cl | CHO | |
| 569 | $CH_3$ | Br | CHO | |
| 570 | $C_2H_5$ | Br | CHO | |
| 571 | n-$C_3H_7$ | Br | CHO | |
| 572 | iso-$C_3H_7$ | Br | CHO | |
| 573 | n-$C_4H_9$ | Br | CHO | |
| 574 | n-$C_{13}H_{17}$ | Br | CHO | |
| 575 | Cyclo-$C_6H_{11}$ | Br | CHO | |
| 576 | $C_6H_5$ | Br | CHO | |
| 577 | $C_2H_4Br$ | Br | CHO | |
| 578 | $NH_2$ | Br | CHO | |
| 579 | $N(CH_3)_2$ | Br | CHO | |
| 580 | $N(C_2H_5)_2$ | Br | CHO | |

Tabelle 5 - Fortsetzung

| Bsp.-Nr. | $L^1$ | $L^2$ | $L^3$ | Schmelzpunkt [°C] |
|---|---|---|---|---|
| 581 | $CH_3$ | Cl | $CH_3$ | |
| 582 | $C_2H_5$ | Cl | $CH_3$ | |
| 583 | $n-C_3H_7$ | Cl | $CH_3$ | |
| 584 | $iso-C_3H_7$ | Cl | $CH_3$ | |
| 585 | $n-C_4H_9$ | Cl | $CH_3$ | |
| 586 | $n-C_{13}H_{17}$ | Cl | $CH_3$ | |
| 587 | $Cyclo-C_6H_{11}$ | Cl | $CH_3$ | |
| 588 | $C_6H_5$ | Cl | $CH_3$ | |
| 589 | $C_2H_4Cl$ | Cl | $CH_3$ | |
| 590 | $NH_2$ | Cl | $CH_3$ | |
| 591 | $N(CH_3)_2$ | Cl | $CH_3$ | |
| 592 | $N(C_2H_5)_2$ | Cl | $CH_3$ | |
| 593 | $CH_3$ | Br | $CH_3$ | |
| 594 | $C_2H_5$ | Br | $CH_3$ | |
| 595 | $n-C_3H_7$ | Br | $CH_3$ | |
| 596 | $iso-C_3H_7$ | Br | $CH_3$ | |
| 597 | $n-C_4H_9$ | Br | $CH_3$ | |
| 598 | $n-C_{13}H_{17}$ | Br | $CH_3$ | |
| 599 | $Cyclo-C_6H_{11}$ | Br | $CH_3$ | |
| 600 | $C_6H_5$ | Br | $CH_3$ | |
| 601 | $C_2H_4Br$ | Br | $CH_3$ | |
| 602 | $NH_2$ | Br | $CH_3$ | |
| 603 | $N(CH_3)_2$ | Br | $CH_3$ | |
| 604 | $N(C_2H_5)_2$ | Br | $CH_3$ | |
| 605 | $CH_3$ | Cl | $C_6H_5$ | |
| 606 | $C_2H_5$ | Cl | $C_6H_5$ | |
| 607 | $n-C_3H_7$ | Cl | $C_6H_5$ | |
| 608 | $iso-C_3H_7$ | Cl | $C_6H_5$ | |
| 609 | $n-C_4H_9$ | Cl | $C_6H_5$ | |
| 610 | $n-C_{13}H_{17}$ | Cl | $C_6H_5$ | |
| 611 | $Cyclo-C_6H_{11}$ | Cl | $C_6H_5$ | |
| 612 | $C_6H_5$ | Cl | $C_6H_5$ | |

Tabelle 5 - Fortsetzung

| Bsp.-Nr. | $L^1$ | $L^2$ | $L^3$ | Schmelzpunkt [°C] |
|---|---|---|---|---|
| 613 | $C_2H_4Cl$ | Cl | $C_6H_5$ | |
| 614 | $NH_2$ | Cl | $C_6H_5$ | |
| 615 | $N(CH_3)_2$ | Cl | $C_6H_5$ | |
| 616 | $N(C_2H_5)_2$ | Cl | $C_6H_5$ | |
| 617 | $CH_3$ | Br | $C_6H_5$ | |
| 618 | $C_2H_5$ | Br | $C_6H_5$ | |
| 619 | n-$C_3H_7$ | Br | $C_6H_5$ | |
| 620 | iso-$C_3H_7$ | Br | $C_6H_5$ | |
| 621 | n-$C_4H_9$ | Br | $C_6H_5$ | |
| 622 | n-$C_{13}H_{17}$ | Br | $C_6H_5$ | |
| 623 | Cyclo-$C_6H_{11}$ | Br | $C_6H_5$ | |
| 624 | $C_6H_5$ | Br | $C_6H_5$ | |
| 625 | $C_2H_4Br$ | Br | $C_6H_5$ | |
| 626 | $NH_2$ | Br | $C_6H_5$ | |
| 627 | $N(CH_3)_2$ | Br | $C_6H_5$ | |
| 628 | $N(C_2H_5)_2$ | Br | $C_6H_5$ | |
| 629 | $CH_3$ | Cl | H | |
| 630 | $C_2H_5$ | Cl | H | |
| 631 | n-$C_3H_7$ | Cl | H | |
| 632 | iso-$C_3H_7$ | Cl | H | |
| 633 | n-$C_4H_9$ | Cl | H | |
| 634 | n-$C_{13}H_{17}$ | Cl | H | |
| 635 | Cyclo-$C_6H_{11}$ | Cl | H | |
| 636 | $C_6H_5$ | Cl | H | |
| 637 | $C_2H_4OH$ | Cl | H | |
| 638 | $NH_2$ | Cl | H | |
| 639 | $N(CH_3)_2$ | Cl | H | |
| 640 | $N(C_2H_5)_2$ | Cl | H | |
| 641 | $CH_3$ | Br | H | |
| 642 | $C_2H_5$ | Br | H | |
| 643 | n-$C_3H_7$ | Br | H | |
| 644 | iso-$C_3H_7$ | Br | H | |

Tabelle 5 - Fortsetzung

| Bsp.-Nr. | $L^1$ | $L^2$ | $L^3$ | Schmelzpunkt [°C] |
|---|---|---|---|---|
| 645 | $n-C_4H_9$ | Br | H | |
| 646 | $n-C_{13}H_{17}$ | Br | H | |
| 647 | Cyclo-$C_6H_{11}$ | Br | H | |
| 648 | $C_6H_5$ | Br | H | |
| 649 | $C_2H_4Br$ | Br | H | |
| 650 | $NH_2$ | Br | H | |
| 651 | $N(CH_3)_2$ | Br | H | |
| 652 | $N(C_2H_5)_2$ | Br | H | |

Beispiel 653

4-Chlor-3-cyano-2-dimethylaminoformamidino-1-methylpyrrol-5-carbaldehydoxim

7,2 g 4-Chlor-3-cyano-2-dimethylaminoformamidino-5-formyl-1-methylpyrrol (Beispiel 143), 4,2 g Hydroxylammoniumchlorid und 5,1 g Natriumhydrogencarbonat wurden in 100 ml Ethanol/Wasser (1:1) 3 Stunden unter Rückfluß erhitzt. Nach dem Abkühlen wurde mit 0,5 l Wasser versetzt und der Niederschlag abgesaugt, nachgewaschen und getrocknet. Man erhielt 5,5 g der Verbindung der Formel

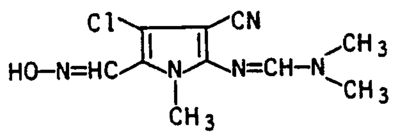

vom Schmelzpunkt 220 bis 222 °C. IR-, NMR-, UV- und Massenspektren und die Elementaranalyse stehen mit der Konstitutionsformel im Einklang.

Analog Beispiel 653 werden die in Tabelle 6 aufgeführten Verbindungen erhalten.

Tabelle 6

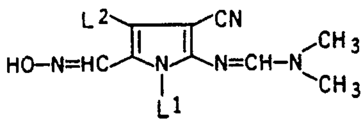

| Bsp.-Nr. | $L^1$ | $L^2$ | Schmelzpunkt [°C] |
|---|---|---|---|
| 654 | $C_2H_5$ | Cl | |
| 655 | $n-C_3H_7$ | Cl | |
| 656 | $iso-C_3H_7$ | Cl | |
| 657 | $n-C_4H_9$ | Cl | |
| 658 | $iso-C_4H_9$ | Cl | |
| 659 | $sec-C_4H_9$ | Cl | |
| 660 | $n-C_5H_{11}$ | Cl | |
| 661 | $Neo-C_5H_{11}$ | Cl | |
| 662 | $n-C_6H_{13}$ | Cl | |
| 663 | $n-C_7H_{15}$ | Cl | |
| 664 | $n-C_8H_{17}$ | Cl | |
| 665 | $n-C_9H_{19}$ | Cl | |
| 666 | $n-C_{10}H_{21}$ | Cl | |
| 667 | $n-C_{11}H_{23}$ | Cl | |
| 668 | $n-C_{12}H_{25}$ | Cl | |
| 669 | $n-C_{13}H_{27}$ | Cl | |
| 670 | $n-C_{14}H_{29}$ | Cl | |
| 671 | $n-C_{15}H_{31}$ | Cl | |
| 672 | $n-C_{16}H_{33}$ | Cl | |
| 673 | $n-C_{17}H_{35}$ | Cl | |
| 674 | $n-C_{18}H_{37}$ | Cl | |
| 675 | $n-C_{19}H_{39}$ | Cl | |
| 676 | $n-C_{20}H_{41}$ | Cl | |
| 677 | $Cyclo-C_3H_5$ | Cl | |
| 678 | $Cyclo-C_4H_7$ | Cl | |
| 679 | $Cyclo-C_5H_9$ | Cl | |
| 680 | $Cyclo-C_6H_{11}$ | Cl | |
| 681 | $Cyclo-C_7H_{13}$ | Cl | |
| 682 | $Cyclo-C_8H_{15}$ | Cl | |
| 683 | $Cyclo-C_9H_{17}$ | Cl | |
| 684 | $Cyclo-C_{10}H_{19}$ | Cl | |
| 685 | $CH_2CH_2Cl$ | Cl | |

Tabelle 6 - Fortsetzung

| Bsp.-Nr. | $L^1$ | $L^2$ | Schmelzpunkt [°C] |
|---|---|---|---|
| 686 | $CH_2CH_2OCH_3$ | Cl | |
| 687 | $CH_2CH_2OC_2H_5$ | Cl | |
| 688 | $CH_2CH_2N{=}CH{-}N(CH_3)_2$ | Cl | |
| 689 | $CH_2CH_2N(CH_3)_2$ | Cl | |
| 690 | $CH_2CH_2N(C_2H_5)_2$ | Cl | |
| 691 | $CH_2CH_2SCH_3$ | Cl | |
| 692 | $CH_2CH_2SC_2H_5$ | Cl | |
| 693 | $(CH_2)_3Cl$ | Cl | |
| 694 | $(CH_2)_3OCH_3$ | Cl | |
| 695 | $(CH_2)_3OC_2H_5$ | Cl | |
| 696 | $(CH_2)_3N{=}CH{-}N(CH_3)_2$ | Cl | |
| 697 | $(CH_2)_3N(CH_3)_2$ | Cl | |
| 698 | $(CH_2)_3N(C_2H_5)_2$ | Cl | |
| 699 | $(CH_2)_3SCH_3$ | Cl | |
| 700 | $(CH_2)_3SC_2H_5$ | Cl | |
| 701 | $CH_2C_6H_5$ | Cl | |
| 702 | $CH_2CH_2C_6H_5$ | Cl | |
| 703 | $CH(CH_3)C_6H_5$ | Cl | |
| 704 | $CH(C_6H_5)_2$ | Cl | |
| 705 | $C_6H_5$ | Cl | |
| 706 | $C_6H_4$-(2)-Cl | Cl | |
| 707 | $C_6H_4$-(3)-Cl | Cl | |
| 708 | $C_6H_4$-(4)-Cl | Cl | |
| 709 | $C_6H_4$-(2)-$OCH_3$ | Cl | |
| 710 | $C_6H_4$-(3)-$OCH_3$ | Cl | |
| 711 | $C_6H_4$-(4)-$OCH_3$ | Cl | |
| 712 | $C_6H_4$-(2)-$OC_2H_5$ | Cl | |
| 713 | $C_6H_4$-(3)-$OC_2H_5$ | Cl | |
| 714 | $C_6H_4$-(4)-$OC_2H_5$ | Cl | |
| 715 | $C_6H_4$-(2)-$NO_2$ | Cl | |
| 716 | $C_6H_4$-(3)-$NO_2$ | Cl | |
| 717 | $C_6H_4$-(4)-$NO_2$ | Cl | |

Tabelle 6 - Fortsetzung

| Bsp.-Nr. | L1 | L2 | Schmelzpunkt [°C] |
|---|---|---|---|
| 718 | $CH_2-COOCH_3$ | Cl | |
| 719 | $CH_2-COOC_2H_5$ | Cl | |
| 720 | $N=CH-N(CH_3)_2$ | Cl | |
| 721 | $N(CH_3)_2$ | Cl | |
| 722 | $N(C_2H_5)_2$ | Cl | |
| 723 | $CH_3$ | Br | |
| 724 | $C_2H_5$ | Br | |
| 725 | $n-C_3H_7$ | Br | |
| 726 | $iso-C_3H_7$ | Br | |
| 727 | $CH_2CH_2NH_2$ | Br | |
| 728 | $CH_2CH_2CH_2NH_2$ | Br | |
| 729 | $Cyclo-C_6H_{11}$ | Br | |
| 730 | $C_6H_5$ | Br | |
| 731 | $C_2H_4Br$ | Br | |
| 732 | $NH_2$ | Br | |
| 733 | $N(CH_3)_2$ | Br | |
| 734 | $N(C_2H_5)_2$ | Br | |

Beispiel 735

2-Acetylamino-4-chlor-3,5-dicyano-1-methylpyrrol

5,0 g 4-Chlor-3-cyano-2-dimethylaminoformamidino-1-methylpyrrol-5-carbaldehydoxim (Beispiel 653) wurden in 40 ml Acetanhydrid 15 Stunden unter Rückfluß erhitzt. Nach Zugabe von 100 ml Wasser wurde weitere 10 Minuten unter Rückfluß erhitzt und das Reaktionsgemisch anschließend auf 300 g Eis gegossen. Die Mischung wurde mit halbkonz. Natronlauge auf pH 5 eingestellt und der Niederschlag nach zweistündigem Stehen bei Raumtemperatur abgesaugt, nachgewaschen und getrocknet. Man erhielt 2,2 g (47 % d. Th.) der Verbindung der Formel

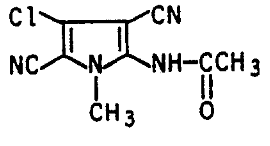

vom Schmelzpunkt 180 bis 182 °C. IR-, NMR-, UV- und Massenspektren und die Elementaranalyse stehen mit der Konstitutionsformel im Einklang.

Analog Beispiel 735 werden die in Tabelle 7 aufgeführten Verbindungen erhalten.

Tabelle 7

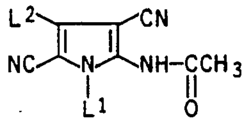

| Bsp.-Nr. | $L^1$ | $L^2$ | Schmelzpunkt [°C] |
|---|---|---|---|
| 736 | $C_2H_5$ | Cl | |
| 737 | n-$C_3H_7$ | Cl | |
| 738 | iso-$C_3H_7$ | Cl | |
| 739 | n-$C_4H_9$ | Cl | |
| 740 | iso-$C_4H_9$ | Cl | |
| 741 | sec-$C_4H_9$ | Cl | |
| 742 | n-$C_5H_{11}$ | Cl | |
| 743 | Neo-$C_5H_{11}$ | Cl | |
| 744 | n-$C_6H_{13}$ | Cl | |
| 745 | n-$C_7H_{15}$ | Cl | |
| 746 | n-$C_8H_{17}$ | Cl | |
| 747 | n-$C_9H_{19}$ | Cl | |
| 748 | n-$C_{10}H_{21}$ | Cl | |
| 749 | n-$C_{11}H_{23}$ | Cl | |
| 750 | n-$C_{12}H_{25}$ | Cl | |
| 751 | n-$C_{13}H_{27}$ | Cl | |
| 752 | n-$C_{14}H_{29}$ | Cl | |
| 753 | n-$C_{15}H_{31}$ | Cl | |
| 754 | n-$C_{16}H_{33}$ | Cl | |
| 755 | n-$C_{17}H_{35}$ | Cl | |
| 756 | n-$C_{18}H_{37}$ | Cl | |
| 757 | n-$C_{19}H_{39}$ | Cl | |
| 758 | n-$C_{20}H_{41}$ | Cl | |
| 759 | Cyclo-$C_3H_5$ | Cl | |
| 760 | Cyclo-$C_4H_7$ | Cl | |
| 761 | Cyclo-$C_5H_9$ | Cl | |
| 762 | Cyclo-$C_6H_{11}$ | Cl | |
| 763 | Cyclo-$C_7H_{13}$ | Cl | |
| 764 | Cyclo-$C_8H_{15}$ | Cl | |

Tabelle 7 - Fortsetzung

| Bsp.-Nr. | $L^1$ | $L^2$ | Schmelzpunkt [°C] |
|---|---|---|---|
| 765 | Cyclo-$C_9H_{17}$ | Cl | |
| 766 | Cyclo-$C_{10}H_{19}$ | Cl | |
| 767 | $CH_2CH_2Cl$ | Cl | |
| 768 | $CH_2CH_2OCH_3$ | Cl | |
| 769 | $CH_2CH_2OC_2H_5$ | Cl | |
| 770 | $CH_2CH_2N{=}CH{-}N(CH_3)_2$ | Cl | |
| 771 | $CH_2CH_2N(CH_3)_2$ | Cl | |
| 772 | $CH_2CH_2N(C_2H_5)_2$ | Cl | |
| 773 | $CH_2CH_2SCH_3$ | Cl | |
| 774 | $CH_2CH_2SC_2H_5$ | Cl | |
| 775 | $(CH_2)_3Cl$ | Cl | |
| 776 | $(CH_2)_3OCH_3$ | Cl | |
| 777 | $(CH_2)_3OC_2H_5$ | Cl | |
| 778 | $(CH_2)_3N{=}CH{-}N(CH_3)_2$ | Cl | |
| 779 | $(CH_2)_3N(CH_3)_2$ | Cl | |
| 780 | $(CH_2)_3N(C_2H_5)_2$ | Cl | |
| 781 | $(CH_2)_3SCH_3$ | Cl | |
| 782 | $(CH_2)_3SC_2H_5$ | Cl | |
| 783 | $CH_2C_6H_5$ | Cl | |
| 784 | $CH_2CH_2C_6H_5$ | Cl | |
| 785 | $CH(CH_3)C_6H_5$ | Cl | |
| 786 | $CH(C_6H_5)_2$ | Cl | |
| 787 | $C_6H_5$ | Cl | |
| 788 | $C_6H_4$-(2)-Cl | Cl | |
| 789 | $C_6H_4$-(3)-Cl | Cl | |
| 790 | $C_6H_4$-(4)-Cl | Cl | |
| 791 | $C_6H_4$-(2)-$OCH_3$ | Cl | |
| 792 | $C_6H_4$-(3)-$OCH_3$ | Cl | |
| 793 | $C_6H_4$-(4)-$OCH_3$ | Cl | |
| 794 | $C_6H_4$-(2)-$OC_2H_5$ | Cl | |
| 795 | $C_6H_4$-(3)-$OC_2H_5$ | Cl | |
| 796 | $C_6H_4$-(4)-$OC_2H_5$ | Cl | |

Tabelle 7 - Fortsetzung

| Bsp.-Nr. | $L^1$ | $L^2$ | Schmelzpunkt [°C] |
|---|---|---|---|
| 797 | $C_6H_4$-(2)-$NO_2$ | Cl | |
| 798 | $C_6H_4$-(3)-$NO_2$ | Cl | |
| 799 | $C_6H_4$-(4)-$NO_2$ | Cl | |
| 800 | $CH_2$-$COOCH_3$ | Cl | |
| 801 | $CH_2$-$COOC_2H_5$ | Cl | |
| 802 | N=CH-N$(CH_3)_2$ | Cl | |
| 803 | $N(CH_3)_2$ | Cl | |
| 804 | $N(C_2H_5)_2$ | Cl | |
| 805 | $CH_3$ | Br | |
| 806 | $C_2H_5$ | Br | |
| 807 | n-$C_3H_7$ | Br | |
| 808 | iso-$C_3H_7$ | Br | |
| 809 | n-$C_4H_9$ | Br | |
| 810 | n-$C_{13}H_{17}$ | Br | |
| 811 | Cyclo-$C_6H_{11}$ | Br | |
| 812 | $C_6H_5$ | Br | |
| 813 | $C_2H_4Br$ | Br | |
| 814 | $N(CH_3)_2$ | Br | |
| 815 | $N(C_2H_5)_2$ | Br | |

Beispiel 816

4-Chlor-3,5-dicyano-2-dimethylformamidino-1-methylpyrrol

4,8 g 4-Chlor-3-cyano-2-dimethylaminoformamidino-5-formyl-1-methylpyrrol (Beispiel 143) und 1,8 g Kupfer(I)cyanid wurden in 50 ml DMF 5 Tage unter Rückfluß erhitzt. Nach dem Abkühlen wurde die Reaktionsmischung filtriert und das Filtrat mit 200 ml Wasser versetzt. Der Niederschlag wurde abgesaugt, nachgewaschen und getrocknet. Man erhielt 1,4 g (30 %) der Verbindung der Formel

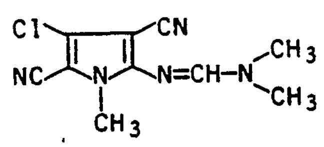

vom Schmelzpunkt 180 bis 184 °C. IR-, NMR-, UV- und Massenspektren und die Elementaranalyse stehen mit der Konstitutionsformel im Einklang.

Analog Beispiel 816 werden die in Tabelle 8 aufgeführten Verbindungen erhalten.

Tabelle 8

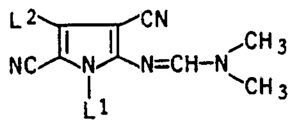

| Bsp.-Nr. | $L^1$ | $L^2$ | Schmelzpunkt [°C] |
|---|---|---|---|
| 816 | $CH_3$ | Cl | |
| 817 | $C_2H_5$ | Cl | |
| 818 | $n-C_3H_7$ | Cl | |
| 819 | $iso-C_3H_7$ | Cl | |
| 820 | $n-C_4H_9$ | Cl | |
| 821 | $iso-C_4H_9$ | Cl | |
| 822 | $sec-C_4H_9$ | Cl | |
| 823 | $n-C_5H_{11}$ | Cl | |
| 824 | $Neo-C_5H_{11}$ | Cl | |
| 825 | $n-C_6H_{13}$ | Cl | |
| 826 | $n-C_7H_{15}$ | Cl | |
| 827 | $n-C_8H_{17}$ | Cl | |
| 828 | $n-C_9H_{19}$ | Cl | |
| 829 | $n-C_{10}H_{21}$ | Cl | |
| 830 | $n-C_{11}H_{23}$ | Cl | |
| 831 | $n-C_{12}H_{25}$ | Cl | |
| 832 | $n-C_{13}H_{27}$ | Cl | |
| 833 | $n-C_{14}H_{29}$ | Cl | |
| 834 | $n-C_{15}H_{31}$ | Cl | |
| 835 | $n-C_{16}H_{33}$ | Cl | |
| 836 | $n-C_{17}H_{35}$ | Cl | |
| 837 | $n-C_{18}H_{37}$ | Cl | |
| 838 | $n-C_{19}H_{39}$ | Cl | |
| 839 | $n-C_{20}H_{41}$ | Cl | |
| 840 | $Cyclo-C_3H_5$ | Cl | |
| 841 | $Cyclo-C_4H_7$ | Cl | |
| 842 | $Cyclo-C_5H_9$ | Cl | |
| 843 | $Cyclo-C_6H_{11}$ | Cl | |
| 844 | $Cyclo-C_7H_{13}$ | Cl | |

Tabelle 8 - Fortsetzung

| Bsp.-Nr. | L1 | L2 | Schmelzpunkt [°C] |
|---|---|---|---|
| 845 | Cyclo-$C_8H_{15}$ | Cl | |
| 846 | Cyclo-$C_9H_{17}$ | Cl | |
| 847 | Cyclo-$C_{10}H_{19}$ | Cl | |
| 848 | $CH_2-C_6H_5$ | Cl | |
| 849 | $CH_2CH_2C_6H_5$ | Cl | |
| 850 | $CH(CH_3)_2C_6H_5$ | Cl | |
| 851 | $CH(C_6H_5)_2$ | Cl | |
| 852 | $C_6H_5$ | Cl | |

Beispiel 853

5-Acetyl-2-acetylamino-4-acetyloxy-3-cyano-1-methylpyrrol

4,11 g 2-Amino-3-cyano-1-methylpyrrolin-4-on (Beispiel 1) und 4,92 g Natriumacetat wurden in 50 ml Acetanhydrid eine Stunde unter Rückfluß erhitzt. Nach Zugabe von 150 ml Wasser wurde 30 Minuten erneut aufgekocht und dann auf Raumtemperatur abgekühlt. Der Niederschlag wurde abgesaugt, nachgewaschen und getrocknet. Man erhielt 6,7 g (85 % d. Th.) der Verbindung der Formel

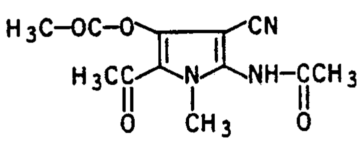

vom Schmelzpunkt 140 bis 141 °C. IR-, NMR-, UV- und Massenspektren und die Elementaranalyse stehen mit der Konstitutionsformel im Einklang.

Analog Beispiel 853 werden die in Tabelle 9 aufgeführten Verbindungen erhalten.

Tabelle 9

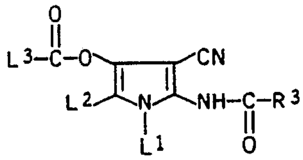

| Bsp.-Nr. | $L^1$ | $L^2$ | $L^3$ | Schmelzpunkt [°C] |
|---|---|---|---|---|
| 853 | $CH_3$ | $COCH_3$ | $CH_3$ | 140-141 |
| 854 | $C_2H_5$ | $COCH_3$ | $CH_3$ | |
| 855 | n-$C_3H_7$ | $COCH_3$ | $CH_3$ | |
| 856 | iso-$C_3H_7$ | $COCH_3$ | $CH_3$ | |
| 857 | n-$C_4H_9$ | $COCH_3$ | $CH_3$ | |
| 858 | iso-$C_4H_9$ | $COCH_3$ | $CH_3$ | |
| 859 | sec-$C_4H_9$ | $COCH_3$ | $CH_3$ | |
| 860 | n-$C_5H_{11}$ | $COCH_3$ | $CH_3$ | |
| 861 | Neo-$C_5H_{11}$ | $COCH_3$ | $CH_3$ | |
| 862 | n-$C_6H_{13}$ | $COCH_3$ | $CH_3$ | |
| 863 | n-$C_7H_{15}$ | $COCH_3$ | $CH_3$ | |
| 864 | n-$C_8H_{17}$ | $COCH_3$ | $CH_3$ | |
| 865 | n-$C_9H_{19}$ | $COCH_3$ | $CH_3$ | |
| 866 | n-$C_{10}H_{21}$ | $COCH_3$ | $CH_3$ | |
| 867 | n-$C_{11}H_{23}$ | $COCH_3$ | $CH_3$ | |
| 868 | n-$C_{12}H_{25}$ | $COCH_3$ | $CH_3$ | |
| 869 | n-$C_{13}H_{27}$ | $COCH_3$ | $CH_3$ | |
| 870 | n-$C_{14}H_{29}$ | $COCH_3$ | $CH_3$ | |
| 871 | n-$C_{15}H_{31}$ | $COCH_3$ | $CH_3$ | |
| 872 | n-$C_{16}H_{33}$ | $COCH_3$ | $CH_3$ | |
| 873 | n-$C_{17}H_{35}$ | $COCH_3$ | $CH_3$ | |
| 874 | n-$C_{18}H_{37}$ | $COCH_3$ | $CH_3$ | |
| 875 | n-$C_{19}H_{39}$ | $COCH_3$ | $CH_3$ | |
| 876 | n-$C_{20}H_{41}$ | $COCH_3$ | $CH_3$ | |
| 877 | $CH_2CH{=}CH_2$ | $COCH_3$ | $CH_3$ | |
| 878 | $CH_2C{\equiv}CH$ | $COCH_3$ | $CH_3$ | |
| 879 | Cyclo-$C_3H_5$ | $COCH_3$ | $CH_3$ | |
| 880 | Cyclo-$C_4H_7$ | $COCH_3$ | $CH_3$ | |
| 881 | Cyclo-$C_6H_{11}$ | $COCH_3$ | $CH_3$ | |
| 882 | Cyclo-$C_7H_{13}$ | $COCH_3$ | $CH_3$ | |
| 883 | Cyclo-$C_8H_{15}$ | $COCH_3$ | $CH_3$ | |
| 884 | Cyclo-$C_9H_{17}$ | $COCH_3$ | $CH_3$ | |

Tabelle 9 - Fortsetzung

| Bsp.-Nr. | $L^1$ | $L^2$ | $L^3$ | Schmelzpunkt [°C] |
|---|---|---|---|---|
| 885 | Cyclo-$C_{10}H_{19}$ | $COCH_3$ | $CH_3$ | |
| 886 | $CH_2CH_2OCOCH_3$ | $COCH_3$ | $CH_3$ | |
| 887 | $CH_2CH_2OCH_3$ | $COCH_3$ | $CH_3$ | |
| 888 | $CH_2CH_2OC_2H_5$ | $COCH_3$ | $CH_3$ | |
| 889 | $CH_2CH_2NHCOCH_3$ | $COCH_3$ | $CH_3$ | |
| 890 | $CH_2CH_2N(CH_3)_2$ | $COCH_3$ | $CH_3$ | |
| 891 | $CH_2CH_2N(C_2H_5)_2$ | $COCH_3$ | $CH_3$ | |
| 892 | $CH_2CH_2SCH_3$ | $COCH_3$ | $CH_3$ | |
| 893 | $CH_2CH_2SC_2H_5$ | $COCH_3$ | $CH_3$ | |
| 894 | $(CH_2)_3OCOCH_3$ | $COCH_3$ | $CH_3$ | |
| 895 | $(CH_2)_3OCH_3$ | $COCH_3$ | $CH_3$ | |
| 896 | $(CH_2)_3OC_2H_5$ | $COCH_3$ | $CH_3$ | |
| 897 | $(CH_2)_3NHCOCH_3$ | $COCH_3$ | $CH_3$ | |
| 898 | $(CH_2)_3N(CH_3)_2$ | $COCH_3$ | $CH_3$ | |
| 899 | $(CH_2)_3N(C_2H_5)_2$ | $COCH_3$ | $CH_3$ | |
| 900 | $(CH_2)_3SCH_3$ | $COCH_3$ | $CH_3$ | |
| 901 | $(CH_2)_3SC_2H_5$ | $COCH_3$ | $CH_3$ | |
| 902 | $CH_2C_6H_5$ | $COCH_3$ | $CH_3$ | |
| 903 | $CH_2CH_2C_6H_5$ | $COCH_3$ | $CH_3$ | |
| 904 | $CH(CH_3)C_6H_5$ | $COCH_3$ | $CH_3$ | |
| 905 | $CH(C_6H_5)_2$ | $COCH_3$ | $CH_3$ | |
| 906 | $C_6H_5$ | $COCH_3$ | $CH_3$ | |
| 907 | $C_6H_4$-(2)-Cl | $COCH_3$ | $CH_3$ | |
| 908 | $C_6H_4$-(3)-Cl | $COCH_3$ | $CH_3$ | |
| 909 | $C_6H_4$-(4)-Cl | $COCH_3$ | $CH_3$ | |
| 910 | $C_6H_4$-(2)-$NO_2$ | $COCH_3$ | $CH_3$ | |
| 911 | $C_6H_4$-(3)-$NO_2$ | $COCH_3$ | $CH_3$ | |
| 912 | $C_6H_4$-(4)-$NO_2$ | $COCH_3$ | $CH_3$ | |
| 913 | $C_6H_4$-(2)-CN | $COCH_3$ | $CH_3$ | |
| 914 | $C_6H_4$-(3)-CN | $COCH_3$ | $CH_3$ | |
| 915 | $C_6H_4$-(4)-CN | $COCH_3$ | $CH_3$ | |
| 916 | $C_6H_4$-(2)-$COOCH_3$ | $COCH_3$ | $CH_3$ | |

Tabelle 9 - Fortsetzung

| Bsp.-Nr. | $L^1$ | $L^2$ | $L^3$ | Schmelzpunkt [°C] |
|---|---|---|---|---|
| 917 | $C_6H_4$-(3)-$COOCH_3$ | $COCH_3$ | $CH_3$ | |
| 918 | $C_6H_4$-(4)-$COOCH_3$ | $COCH_3$ | $CH_3$ | |
| 919 | Pyrid-2-yl | $COCH_3$ | $CH_3$ | |
| 920 | Pyrid-3-yl | $COCH_3$ | $CH_3$ | |
| 921 | Pyrid-4-yl | $COCH_3$ | $CH_3$ | |
| 922 | $CH_2$-$COOCH_3$ | $COCH_3$ | $CH_3$ | |
| 923 | $CH_2$-$COOC_2H_5$ | $COCH_3$ | $CH_3$ | |
| 924 | $NHCOCH_3$ | $COCH_3$ | $CH_3$ | |
| 925 | $N(CH_3)_2$ | $COCH_3$ | $CH_3$ | |
| 926 | $N(C_2H_5)_2$ | $COCH_3$ | $CH_3$ | |
| 927 | $CH_3$ | $CH_3$ | $CH_3$ | |
| 928 | $C_2H_5$ | $CH_3$ | $CH_3$ | |
| 929 | iso-$C_3H_7$ | $CH_3$ | $CH_3$ | |
| 930 | $CH_2CH$=$CH_2$ | $CH_3$ | $CH_3$ | |
| 931 | Cyclo-$C_6H_{11}$ | $CH_3$ | $CH_3$ | |
| 932 | $CH_2CH_2OCOCH_3$ | $CH_3$ | $CH_3$ | |
| 933 | $CH_2CH_2OCH_3$ | $CH_3$ | $CH_3$ | |
| 934 | $C_6H_5$ | $CH_3$ | $CH_3$ | |
| 935 | Pyrid-3-yl | $CH_3$ | $CH_3$ | |
| 936 | $NHCOCH_3$ | $CH_3$ | $CH_3$ | |
| 937 | $CH_3$ | $C_6H_5$ | $CH_3$ | |
| 938 | $C_2H_5$ | $C_6H_5$ | $CH_3$ | |
| 939 | iso-$C_3H_7$ | $C_6H_5$ | $CH_3$ | |
| 940 | $CH_2CH$=$CH_2$ | $C_6H_5$ | $CH_3$ | |
| 941 | $CH_2CH_2OCOCH_3$ | $C_6H_5$ | $CH_3$ | |
| 942 | $CH_2CH_2OCH_3$ | $C_6H_5$ | $CH_3$ | |
| 943 | $C_6H_5$ | $C_6H_5$ | $CH_3$ | |
| 944 | $C_6H_4$-(4)-CN | $C_6H_5$ | $CH_3$ | |
| 945 | $NHCOCH_3$ | $C_6H_5$ | $CH_3$ | |
| 946 | $(CH_2)_3$-O-$CH_2C_6H_5$ | $COCH_3$ | $CH_3$ | |
| 947 | $(CH_2)_3$-O-$(CH_2)_2$-O--$C_6H_5$ | $COCH_3$ | $CH_3$ | |

Tabelle 9 - Fortsetzung

| Bsp.-Nr. | $L^1$ | $L^2$ | $L^3$ | Schmelzpunkt [°C] |
|---|---|---|---|---|
| 948 | $CH_3$ | $COCH(CH_3)_2$ | $CH(CH_3)_2$ | |
| 949 | $CH_3$ | $COC_6H_5$ | $C_6H_5$ | |
| 950 | $CH_3$ | CO-n-$C_6H_{13}$ | n-$C_6H_{13}$ | |

Beispiel 951

4,1 g 2-Amino-3-cyano-1-methylpyrrolin-4-on (Beispiel 1), 4,2 g Hydroxylammoniumchlorid und 5,1 g Natriumhydrogencarbonat wurden 3 Stunden in 100 ml $H_2O$/Etanol (1:1) unter Rückfluß erhitzt. Nach dem Abkühlen auf Raumtemperatur verdünnte man mit $H_2O$ und saugte den Niederschlag ab. Man erhielt 4,2 g (83 % d. Th.) der Verbindung der Formel

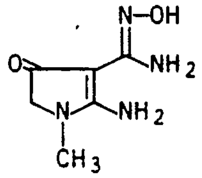

vom Schmelzpunkt > 260 °C. IR-, NMR-, UV- und Massenspektren und die Elementaranalyse stehen mit der Konstitutionsformel im Einklang.

In analoger Weise werden die in der folgenden Tabelle 10 aufgeführten Verbindungen erhalten.

Tabelle 10

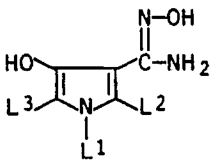

| Bsp.-Nr. | $L^1$ | $L^2$ | $L^3$ | Schmelzpunkt [°C] |
|---|---|---|---|---|
| 952 | $C_2H_5$ | $NH_2$ | H | |
| 953 | n-$C_3H_7$ | $NH_2$ | H | |
| 954 | iso-$C_3H_7$ | $NH_2$ | H | |
| 955 | n-$C_4H_9$ | $NH_2$ | H | |
| 956 | iso-$C_4H_9$ | $NH_2$ | H | |

Tabelle 10 - Fortsetzung

| Bsp.-Nr. | $L^1$ | $L^2$ | $L^3$ | Schmelzpunkt [°C] |
|---|---|---|---|---|
| 957 | sec-$C_4H_9$ | $NH_2$ | H | |
| 958 | n-$C_5H_{11}$ | $NH_2$ | H | |
| 959 | Neo-$C_5H_{11}$ | $NH_2$ | H | |
| 960 | n-$C_6H_{13}$ | $NH_2$ | H | |
| 961 | n-$C_7H_{15}$ | $NH_2$ | H | |
| 962 | n-$C_8H_{17}$ | $NH_2$ | H | |
| 963 | n-$C_9H_{19}$ | $NH_2$ | H | |
| 964 | n-$C_{10}H_{21}$ | $NH_2$ | H | |
| 965 | n-$C_{11}H_{23}$ | $NH_2$ | H | |
| 966 | n-$C_{12}H_{25}$ | $NH_2$ | H | |
| 967 | n-$C_{13}H_{27}$ | $NH_2$ | H | |
| 968 | n-$C_{14}H_{29}$ | $NH_2$ | H | |
| 969 | n-$C_{15}H_{31}$ | $NH_2$ | H | |
| 970 | n-$C_{16}H_{33}$ | $NH_2$ | H | |
| 971 | n-$C_{17}H_{35}$ | $NH_2$ | H | |
| 972 | n-$C_{18}H_{37}$ | $NH_2$ | H | |
| 973 | n-$C_{19}H_{39}$ | $NH_2$ | H | |
| 974 | n-$C_{20}H_{41}$ | $NH_2$ | H | |
| 975 | $CH_2CH{=}CH_2$ | $NH_2$ | H | |
| 976 | $CH_2C{\equiv}CH$ | $NH_2$ | H | |
| 977 | Cyclo-$C_3H_5$ | $NH_2$ | H | |
| 978 | Cyclo-$C_4H_7$ | $NH_2$ | H | |
| 979 | Cyclo-$C_5H_9$ | $NH_2$ | H | |
| 980 | Cyclo-$C_6H_{11}$ | $NH_2$ | H | |
| 981 | Cyclo-$C_7H_{13}$ | $NH_2$ | H | |
| 982 | Cyclo-$C_8H_{15}$ | $NH_2$ | H | |
| 983 | Cyclo-$C_9H_{17}$ | $NH_2$ | H | |
| 984 | Cyclo-$C_{10}H_{19}$ | $NH_2$ | H | |
| 985 | $CH_2CH_2OH$ | $NH_2$ | H | |
| 986 | $CH_2CH_2OCH_3$ | $NH_2$ | H | |
| 987 | $CH_2CH_2OC_2H_5$ | $NH_2$ | H | |
| 988 | $CH_2CH_2NH_2$ | $NH_2$ | H | |

Tabelle 10 - Fortsetzung

| Bsp.-Nr. | $L^1$ | $L^2$ | $L^3$ | Schmelzpunkt [°C] |
|---|---|---|---|---|
| 989 | $CH_2CH_2N(CH_3)_2$ | $NH_2$ | H | |
| 990 | $CH_2CH_2N(C_2H_5)_2$ | $NH_2$ | H | |
| 991 | $CH_2CH_2SCH_3$ | $NH_2$ | H | |
| 992 | $CH_2CH_2SC_2H_5$ | $NH_2$ | H | |
| 993 | $(CH_2)_3OH$ | $NH_2$ | H | |
| 994 | $(CH_2)_3OCH_3$ | $NH_2$ | H | |
| 995 | $(CH_2)_3OC_2H_5$ | $NH_2$ | H | |
| 996 | $(CH_2)_3NH_2$ | $NH_2$ | H | |
| 997 | $(CH_2)_3N(CH_3)_2$ | $NH_2$ | H | |
| 998 | $(CH_2)_3N(C_2H_5)_2$ | $NH_2$ | H | |
| 999 | $(CH_2)_3SCH_3$ | $NH_2$ | H | |
| 1000 | $(CH_2)_3SC_2H_5$ | $NH_2$ | H | |
| 1001 | $CH_2C_6H_5$ | $NH_2$ | H | 205 (dec) |
| 1002 | $CH_2CH_2C_6H_5$ | $NH_2$ | H | 190 (dec) |
| 1003 | $CH(CH_3)C_6H_5$ | $NH_2$ | H | |
| 1004 | $CH(C_6H_5)_2$ | $NH_2$ | H | |
| 1005 | $C_6H_5$ | $NH_2$ | H | 210 (dec) |
| 1006 | $C_6H_4$-(2)-OH | $NH_2$ | H | |
| 1007 | $C_6H_4$-(3)-OH | $NH_2$ | H | |
| 1008 | $C_6H_4$-(4)-OH | $NH_2$ | H | |
| 1009 | $C_6H_4$-(2)-$OCH_3$ | $NH_2$ | H | |
| 1010 | $C_6H_4$-(3)-$OCH_3$ | $NH_2$ | H | 240 (dec) |
| 1011 | $C_6H_4$-(4)-$OCH_3$ | $NH_2$ | H | 220 (dec) |
| 1012 | $C_6H_4$-(2)-$OC_2H_5$ | $NH_2$ | H | |
| 1013 | $C_6H_4$-(3)-$OC_2H_5$ | $NH_2$ | H | |
| 1014 | $C_6H_4$-(4)-$OC_2H_5$ | $NH_2$ | H | |
| 1015 | $C_6H_4$-(2)-Cl | $NH_2$ | H | |
| 1016 | $C_6H_4$-(3)-Cl | $NH_2$ | H | |
| 1017 | $C_6H_4$-(4)-Cl | $NH_2$ | H | |
| 1018 | $C_6H_4$-(2)-$NO_2$ | $NH_2$ | H | |
| 1019 | $C_6H_4$-(3)-$NO_2$ | $NH_2$ | H | |
| 1020 | $C_6H_4$-(4)-$NO_2$ | $NH_2$ | H | |

Tabelle 10 - Fortsetzung

| Bsp.-Nr. | $L^1$ | $L^2$ | $L^3$ | Schmelzpunkt [°C] |
|---|---|---|---|---|
| 1021 | $C_6H_4$-(2)-$NH_2$ | $NH_2$ | H | |
| 1022 | $C_6H_4$-(3)-$NH_2$ | $NH_2$ | H | |
| 1023 | $C_6H_4$-(4)-$NH_2$ | $NH_2$ | H | |
| 1024 | $C_6H_4$-(2)-$CH_3$ | $NH_2$ | H | 190 (dec) |
| 1025 | $C_6H_4$-(3)-$CH_3$ | $NH_2$ | H | |
| 1026 | $C_6H_4$-(4)-$CH_3$ | $NH_2$ | H | |
| 1027 | $C_6H_4$-(2)-$C_2H_5$ | $NH_2$ | H | |
| 1028 | $C_6H_4$-(3)-$C_2H_5$ | $NH_2$ | H | |
| 1029 | $C_6H_4$-(4)-$C_2H_5$ | $NH_2$ | H | |
| 1030 | $C_6H_4$-(2)-CN | $NH_2$ | H | |
| 1031 | $C_6H_4$-(3)-CN | $NH_2$ | H | |
| 1032 | $C_6H_4$-(4)-CN | $NH_2$ | H | |
| 1033 | $C_6H_4$-(2)-$COOCH_3$ | $NH_2$ | H | |
| 1034 | $C_6H_4$-(3)-$COOCH_3$ | $NH_2$ | H | |
| 1035 | $C_6H_4$-(4)-$COOCH_3$ | $NH_2$ | H | |
| 1036 | Pyrid-2-yl | $NH_2$ | H | |
| 1037 | Pyrid-3-yl | $NH_2$ | H | |
| 1038 | Pyrid-4-yl | $NH_2$ | H | |
| 1039 | $(CH_2)_2$-$C_6H_3$--(3)-Cl-(2)-$CH_3$ | $NH_2$ | H | |
| 1040 | $(CH_2)_2$-$C_6H_4$--(3,4)-$(OCH_3)_2$ | $NH_2$ | H | |
| 1041 | $(CH_2)_2$-$C_6H_3$--(2)-Cl | $NH_2$ | H | |
| 1042 | $C_6H_3$-(3,4)-$Cl_2$ | $NH_2$ | H | |
| 1043 | $CH_2$-$COOCH_3$ | $NH_2$ | H | |
| 1044 | $CH_2$-$COOC_2H_5$ | $NH_2$ | H | |
| 1045 | $NH_2$ | $NH_2$ | H | |
| 1046 | $N(CH_3)_2$ | $NH_2$ | H | |
| 1047 | $N(C_2H_5)_2$ | $NH_2$ | H | |
| 1048 | H | $N(CH_3)_2$ | H | |
| 1049 | H | $N(C_2H_5)_2$ | H | |
| 1050 | H | $N(n\text{-}C_3H_7)_2$ | H | |

Tabelle 10 - Fortsetzung

| Bsp.-Nr. | $L^1$ | $L^2$ | $L^3$ | Schmelzpunkt [°C] |
|---|---|---|---|---|
| 1051 | H | $N(n-C_4H_9)_2$ | H | |
| 1052 | H | 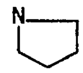 | H | |
| 1053 | H | 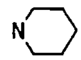 | H | |
| 1054 | H | 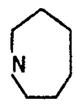 | H | |
| 1055 | H | 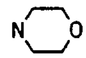 | H | |
| 1056 | H | 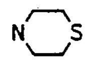 | H | |
| 1057 | H | 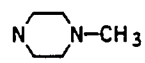 | H | |
| 1058 | $CH_3$ | $NH_2$ | $CH_3$ | |
| 1059 | $C_2H_5$ | $NH_2$ | $CH_3$ | |
| 1060 | iso-$C_3H_7$ | $NH_2$ | $CH_3$ | |
| 1061 | $CH_2CH{=}CH_2$ | $NH_2$ | $CH_3$ | |
| 1062 | Cyclo-$C_6H_{11}$ | $NH_2$ | $CH_3$ | |
| 1063 | $CH_2CH_2OH$ | $NH_2$ | $CH_3$ | |
| 1064 | $CH_2CH_2OCH_3$ | $NH_2$ | $CH_3$ | |
| 1065 | $CH_2C_6H_5$ | $NH_2$ | $CH_3$ | |
| 1066 | $C_6H_5$ | $NH_2$ | $CH_3$ | |
| 1067 | $C_6H_4$-(4)-$OCH_3$ | $NH_2$ | $CH_3$ | |
| 1068 | $C_6H_4$-(2)-$CH_3$ | $NH_2$ | $CH_3$ | |
| 1069 | (3)-Pyridyl | $NH_2$ | $CH_3$ | |
| 1070 | $NH_2$ | $NH_2$ | $CH_3$ | |
| 1071 | H | $N(CH_3)_2$ | $CH_3$ | |
| 1072 | H | $N(C_2H_5)_2$ | $CH_3$ | |
| 1073 | H | 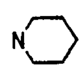 | $CH_3$ | |

Tabelle 10 - Fortsetzung

| Bsp.-Nr. | $L^1$ | $L^2$ | $L^3$ | Schmelzpunkt [°C] |
|---|---|---|---|---|
| 1074 | H | 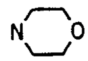 | $CH_3$ | |
| 1075 | $CH_3$ | $NH_2$ | $C_6H_5$ | |
| 1076 | $C_2H_5$ | $NH_2$ | $C_6H_5$ | |
| 1077 | iso-$C_3H_7$ | $NH_2$ | $C_6H_5$ | |
| 1078 | $CH_2CH{=}CH_2$ | $NH_2$ | $C_6H_5$ | |
| 1079 | $CH_2CH_2OH$ | $NH_2$ | $C_6H_5$ | |
| 1080 | $CH_2CH_2OCH_3$ | $NH_2$ | $C_6H_5$ | |
| 1081 | $CH_2C_6H_5$ | $NH_2$ | $C_6H_5$ | |
| 1082 | $C_6H_5$ | $NH_2$ | $C_6H_5$ | |
| 1083 | $C_6H_4$-(4)-$OCH_3$ | $NH_2$ | $C_6H_5$ | |
| 1084 | $C_6H_4$-(4)-CN | $NH_2$ | $C_6H_5$ | |
| 1085 | $NH_2$ | $NH_2$ | $C_6H_5$ | |
| 1086 | H | $N(CH_3)_2$ | $C_6H_5$ | |
| 1087 | H | $N(C_2H_5)_2$ | $C_6H_5$ | |
| 1088 | H | 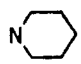 | $C_6H_5$ | |
| 1089 | H | 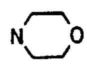 | $C_6H_5$ | |
| 1090 | $(CH_2)_3$-O-$CH_2C_6H_5$ | $NH_2$ | H | |
| 1091 | $(CH_2)_3$-O-$(CH_2)_2$-O--$C_6H_5$ | $NH_2$ | H | |

Beispiel 1092

10,0 g 3-Chlor-4-cyano-5-dimethylaminoformamidino-2-formyl-1-methylpyrrol und 6,1 g Meldrum's Säure wurden in 100 ml Dimethylformamid und 1 ml Piperidin 15 Stunden bei 50 °C gerührt. Nach Zugabe von 200 ml Wasser wurde der Niederschlag abgesaugt und getrocknet. Man erhielt 5,2 g (34 % d.Th.) der Verbindung der Formel

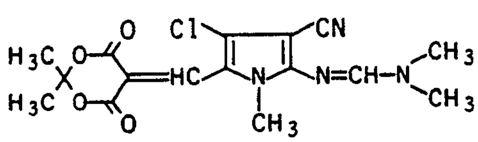

vom Schmelzpunkt 199 °C. IR-, NMR-, UV- und Massenspektren und die Elementaranalyse stehen mit der Konstitutionsformel im Einklang.

Beispiel 1093

6,6 g Malodinitril wurden in 200 ml n-Propanol gelöst. Hierzu wurden 5 g Natriumacetat und 11,9 g 4-Chlor-3-cyano-5-formyl-2-dimethylaminoformamidino-1-methylpyrrol gegeben und das Gemisch wurde 8

Stunden bei Raumtemperatur gerührt. Der Niederschlag wurde abgesaugt, nachgewaschen mit n-Propanol und getrocknet. Man erhielt 13,2 g (92 % d. Th.) der Verbindung der Formel

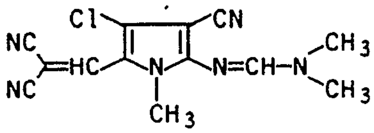

vom Schmelzpunkt 180 °C. IR-, NMR-, UV- und Massenspektren und die Elementaranalyse stehen mit der Konstitutionsformel im Einklang.

In analoger Weise werden die in der folgenden Tabelle 11 aufgeführten Verbindungen erhalten.

Tabelle 11

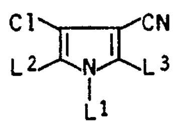

| Bsp.-Nr. | $L^1$ | $L^2$ | $L^3$ | Schmelzpunkt [°C] |
|---|---|---|---|---|
| 1094 | $CH_3$ | $CH=C(COOCH_3)_2$ | $N=CH-N(CH_3)_2$ | 175 |
| 1095 | $C_2H_5$ | $CH=C(COOCH_3)_2$ | $N=CH-N(CH_3)_2$ | |
| 1096 | $C_6H_5$ | $CH=C(COOC_2H_5)_2$ | $N=CH-N(CH_3)_2$ | |
| 1097 | $CH(CH_3)_2$ | $CH=C(COOC_2H_5)_2$ | $NH_2$ | |
| 1098 | $C_2H_5$ | $CH=C(CN)_2$ | $N=CH-N(CH_3)_2$ | |
| 1099 | $n-C_4H_9$ | $CH=C(CN)_2$ | $N=CH-N(CH_3)_2$ | |
| 1100 | $CH_3$ | $CH=C(COOCH_3)(CN)$ | $N=CH-N(CH_3)_2$ | |
| 1101 | $C_6H_5$ | $CH=C(COOC_2H_5)(CN)$ | $NH_2$ | |
| 1102 | $C_6H_5CH_2$ | $CH=C(COOCH_3)(CN)$ | $N=CH-N(CH_3)_2$ | |
| 1103 | $C_6H_5$ | $CH=C(COOCH_3)(CN)$ | $NH_2$ | |

Tabelle 11 - Fortsetzung

| Bsp.-Nr. | $L^1$ | $L^2$ | $L^3$ | Schmelzpunkt [°C] |
|---|---|---|---|---|
| 1104 | $CH_3$ | 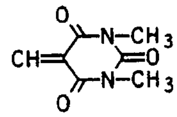 | $N{=}CH{-}N(CH_3)_2$ | |
| 1105 | $C_2H_5$ | 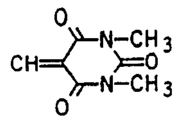 | $NH_2$ | |
| 1106 | $CH_3$ | $CH{=}C(CONHCH_3)(CN)$ | $N{=}CH{-}N(CH_3)_2$ | |
| 1107 | $C_6H_5$ | $CH{=}C(COCH_3)(CN)$ | $NH_2$ | |
| 1108 | Cyclo-$C_6H_{11}$ | $CH{=}C(COC_6H_5)(CN)$ | $N{=}CH{-}N(CH_3)_2$ | |
| 1109 | $CH_3$ | $CH{=}C(C_6H_5)(CN)$ | $N{=}CH{-}N(CH_3)_2$ | |
| 1110 | $CH(CH_3)_2$ | 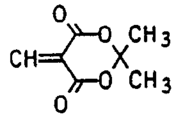 | $N{=}CH{-}N(CH_3)_2$ | |
| 1111 | $CH_3$ | 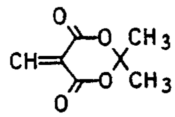 | $NH_2$ | |
| 1112 | $CH(CH_3)_2$ | 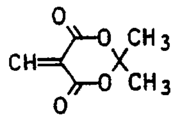 | $NH_2$ | |
| 1113 | $CH_3$ | $CH{=}CH{-}NO_2$ | $N{=}CH{-}N(CH_3)_2$ | |
| 1114 | $CH(CH_3)_2$ | $CH{=}C(CH_3){-}NO_2$ | $N{=}CH{-}N(CH_3)_2$ | |

## Ansprüche

1. Pyrrolderivate der Formel I

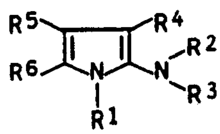

(I)

oder deren Tautomere, in der

$R^1$ Wasserstoff, $C_1$-$C_{20}$-Alkyl, das gegebenenfalls substituiert und/oder durch ein oder mehrere Sauerstoffatome unterbrochen ist, gegebenenfalls substituiertes $C_3$-$C_6$-Alkenyl, gegebenenfalls substituiertes $C_3$-$C_6$-Alkinyl, gegebenenfalls substituiertes $C_3$-$C_{10}$-Cycloalkyl, gegebenenfalls substituiertes Phenyl, Pyridyl, Thienyl, Amino, $C_1$-$C_4$-Alkanoylamino, Benzoylamino, $C_1$-$C_4$-Dialkylamino oder den Rest

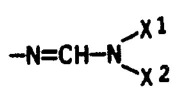

,

wobei $X^1$ für $C_1$-$C_4$-Alkyl oder Phenyl und $X^2$ für $C_1$-$C_4$-Alkyl stehen,

$R^2$ Wasserstoff, $C_1$-$C_4$-Alkyl, Benzyl, $C_3$-$C_6$-Alkenyl, $C_5$-$C_7$-Cycloalkyl oder gegebenenfalls substituiertes

Phenyl,
$R^3$ Wasserstoff, $C_1$-$C_4$-Alkyl, Benzyl oder $C_3$-$C_6$-Alkenyl oder $R^2$ und $R^3$ zusammen einen Rest der Formel

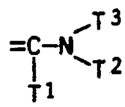 ,

in dem $T^1$ für Wasserstoff, $C_1$-$C_4$-Alkyl oder Phenyl, $T^2$ für $C_1$-$C_4$-Alkyl oder gegebenenfalls substituiertes Phenyl und $T^3$ für $C_1$-$C_4$-Alkyl stehen, oder $R^2$ und $R^3$ zusammen mit dem sie verbindenden Stickstoffatom einen 5- bis 7-gliedrigen gesättigten heterocyclischen Rest, der gegebenenfalls weitere Heteroatome enthält,
$R^4$ Cyano, Carbamoyl, $C_1$-$C_4$-Mono- oder Dialkylcarbamoyl, Thiocarbamoyl, $C_1$-$C_4$-Mono- oder Dialkylthiocarbamoyl oder den Rest

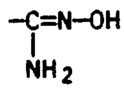 ,

$R^5$ Halogen, Hydroxy, $C_1$-$C_{20}$-Alkanoyloxy oder gegebenenfalls substituiertes Benzoyloxy und
$R^6$ Wasserstoff, $C_1$-$C_4$-Alkyl, gegebenenfalls substituiertes Phenyl, Cyano, Halogen, Nitro, Hydroxysulfonyl, $C_1$-$C_{10}$-Alkanoyl, gegebenenfalls substituiertes Benzoyl oder einen Rest der Formel

$-CH=N-OH$ oder 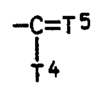 ,

bedeuten, wobei $T^4$ für $C_1$-$C_4$-Alkyl oder gegebenenfalls substituiertes Phenyl und $T^5$ für den Rest einer methylenaktiven Verbindung, Hydroxyimino oder den Rest N-$X^3$ stehen, in dem $X^3$ die Bedeutung von $C_1$-$C_{20}$-Alkyl, das gegebenenfalls substituiert und/oder durch ein oder mehrere Sauerstoffatome unterbrochen ist, gegebenenfalls substituiertem $C_3$-$C_6$-Alkenyl, gegebenenfalls substituiertem $C_3$-$C_6$-Alkinyl, gegebenenfalls substituiertem $C_3$-$C_{10}$-Cycloalkyl, gegebenenfalls substituiertem Phenyl, Pyridyl, $C_1$-$C_4$-Alkoxycarbonylmethyl, Amino, $C_1$-$C_4$-Dialkylamino oder Phenylamino besitzt, mit der Maßgabe, daß $R^1$, $R^2$ und $R^3$ nicht gleichzeitig Wasserstoff bedeuten.

2. Pyrrolderivate gemäß Anspruch 1, dadurch gekennzeichnet, daß
$R^1$ Wasserstoff, $C_1$-$C_{15}$-Alkyl, $C_5$-$C_7$-Cycloalkyl, $C_3$-$C_5$-Alkenyl, $C_2$-$C_5$-Hydroxyalkyl, $C_2$-$C_5$-Halogenalkyl, $C_1$-$C_5$-Alkoxy-$C_2$-$C_5$-alkyl, Phenoxy-$C_2$-$C_5$-alkoxy-$C_2$-$C_5$-alkyl, Phenyl-$C_1$-$C_5$-alkoxy-$C_2$-$C_5$-alkoxy-$C_2$-$C_5$-alkyl, $C_1$-$C_5$-Alkylthio-$C_2$-$C_5$-alkyl, $C_1$-$C_5$-Dialkylamino-$C_2$-$C_5$-alkyl, Phenyl-$C_1$-$C_5$-alkyl, $C_1$-$C_5$-Alkoxycarbonyl-$C_1$-$C_5$-alkyl, einen Rest der Formel $-(CH_2)_n-Y$, in der n für eine Zahl von 1 bis 5 und Y für Pyrrolidino, Piperidino, Morpholino, Thiomorpholino, Piperazino, N-($C_1$-$C_4$-Alkyl)piperazino oder Hexamethylenimino stehen, Phenyl, Pyridyl, Amino oder $C_1$-$C_5$-Dialkylamino,
$R^2$ Wasserstoff, $C_1$-$C_4$-Alkyl, Allyl, Benzyl oder Phenyl,
$R^3$ Wasserstoff, $C_1$-$C_4$-Alkyl, Allyl oder Benzyl oder $R^2$ und $R^3$ zusammen einen Rest der Formel

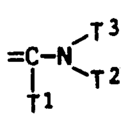 ,

in der $T^1$ für Wasserstoff oder $C_1$-$C_4$-Alkyl, $T^2$ für $C_1$-$C_4$-Alkyl oder Phenyl und $T^3$ für $C_1$-$C_4$-Alkyl stehen, oder $R^2$ und $R^3$ zusammen mit dem sie verbindenden Stickstoffatom Pyrrolidino, Piperidino, Morpholino, Thiomorpholino, Piperazino, N-($C_1$-$C_4$-Alkyl)piperazino oder Hexamethylenimino,
$R^4$ Cyano, Carbamoyl, Thiocarbamoyl oder den Rest der Formel

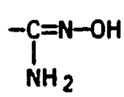 ,

$R^5$ Fluor, Chlor, Brom, Hydroxy, $C_1$-$C_6$-Alkanoyloxy oder Benzoyloxy und
$R^6$ Wasserstoff, $C_1$-$C_4$-Alkyl, Phenyl, $C_1$-$C_6$-Alkanoyl, Benzoyl, Cyano, Chlor, Brom, Nitro, Hydroxysulfonyl oder einen Rest der Formel

–CH=NOH oder 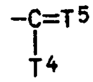 ,

bedeuten, wobei $T^4$ und $T^5$ jeweils die in Anspruch 1 angegebene Bedeutung besitzen.

3. Pyrrolderivate gemäß Anspruch 1, dadurch gekennzeichnet, daß
$R^1$ Wasserstoff, $C_1$-$C_{10}$-Alkyl, $C_3$-$C_4$-Alkenyl, Benzyl, Phenyl oder Amino,
$R^2$ Wasserstoff, $C_1$-$C_4$-Alkyl, Allyl, Benzyl oder Phenyl,
$R^3$ Wasserstoff, $C_1$-$C_4$-Alkyl oder Allyl, oder $R^2$ und $R^3$ zusammen mit dem sie verbindenden Stickstoffatom Pyrrolidino, Piperidino, Morpholino, Thiomorpholino, N-Methylpiperazino oder Hexamethylenimino,
$R^4$ Cyano, Carbamoyl, Thiocarbamoyl oder den Rest der Formel

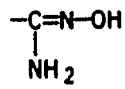 ,

$R^5$ Chlor, Brom, Hydroxy, $C_1$-$C_6$-Alkanoyloxy oder Benzoyloxy und
$R^6$ Wasserstoff, $C_1$-$C_4$-Alkyl, Phenyl, $C_1$-$C_6$-Alkanoyl, Benzoyl, Cyano oder einen Rest der Formel

–CH=NOH , 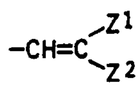 oder CH=N–$X^3$

bedeuten, wobei $Z^1$ und $Z^2$ gleich oder verschieden sind und unabhängig voneinander jeweils für Cyano, $C_1$-$C_4$-Alkoxycarbonyl oder Nitro stehen und $X^3$ die in Anspruch 1 genannte Bedeutung besitzt.

4. Pyrrolverbindungen gemäß Anspruch 1, dadurch gekennzeichnet, daß
$R^1$ $C_1$-$C_4$-Alkyl, Phenyl oder Amino und
$R^2$ und $R^3$ jeweils Wasserstoff oder daß
$R^1$ Wasserstoff,
$R^2$ Methyl, Ethyl, Allyl, Benzyl oder Phenyl,
$R^3$ Methyl, Ethyl oder Allyl oder $R^2$ und $R^3$ zusammen mit dem sie verbindenden Stickstoffatom Pyrrolidino, Piperidino, Morpholino, N-Methylpiperazino oder Hexamethylenimino,
$R^4$ Cyano oder Carbamoyl,
$R^5$ Chlor, Brom oder Hydroxy,
$R^6$ Wasserstoff, Formyl, Cyano oder einen Rest der Formel

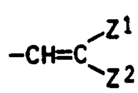

bedeuten, wobei $Z^1$ und $Z^2$ gleich oder verschieden sind und unabhängig voneinander jeweils für Cyano, $C_1$-$C_4$-Alkyl, $C_1$-$C_4$-Alkoxycarbonyl oder Nitro stehen.

5. Verfahren zur Herstellung von Pyrrolderivaten gemäß Anspruch 1, die der Formel Ia oder Ib

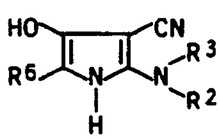 (Ia) 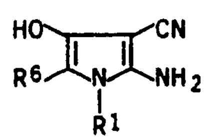 (Ib)

gehorchen, wobei $R^1$, $R^2$, $R^3$ und $R^6$ jeweils die in Anspruch 1 genannte Bedeutung besitzen, dadurch gekennzeichnet, daß man die Verbindung der Formel II

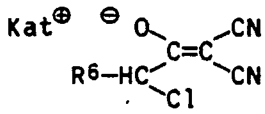

(II),

in der $R^6$ die in Anspruch 1 genannte Bedeutung besitzt und Kat$^\oplus$ ein Kation bedeutet, mit einem Amin der Formel IIIa oder IIIb

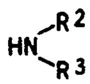

(IIIa)

$R^1{-}NH_2$ . (IIIb)

wobei $R^1$, $R^2$ und $R^3$ jeweils die in Anspruch 1 genannte Bedeutung besitzen, umsetzt.

6. Verwendung der Pyrrolderivate gemäß Anspruch 1 als Diazo- oder Kupplungskomponenten für Azofarbstoffe.